(19) Europäisches Patentamt
European Patent Office
Office européen des brevets

(11) EP 2 657 629 A1

(12) # EUROPEAN PATENT APPLICATION
published in accordance with Art. 153(4) EPC

(43) Date of publication:
**30.10.2013 Bulletin 2013/44**

(21) Application number: **11850663.3**

(22) Date of filing: **21.12.2011**

(51) Int Cl.:
***F25D 3/00*** *(2006.01)* ***A41D 13/00*** *(2006.01)*

(86) International application number:
**PCT/JP2011/079611**

(87) International publication number:
**WO 2012/086676 (28.06.2012 Gazette 2012/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2010 JP 2010288725**
**14.06.2011 JP 2011132056**

(71) Applicant: **Eikan Shoji Co., Ltd.**
**Fukui City, Fukui 910-0019 (JP)**

(72) Inventor: **HASEGAWA, Takashi**
**Fukui-shi**
**Fukui 910-0019 (JP)**

(74) Representative: **Geyer, Fehners & Partner**
**Patentanwälte**
**Perhamerstrasse 31**
**80687 München (DE)**

(54) **BODY TEMPERATURE REGULATION PACK AND PACK HOLDER FOR ATTACHING BODY TEMPERATURE REGULATION PACK**

(57) Provided is a body temperature regulation pack which uses a solid-liquid phase change material to prevent body temperature increase or body temperature decrease of a wearer. When the body temperature regulation pack is arranged on the inner side of a pack holder to be worn under a clothing, the body temperature regulation pack may be bent along a body shape even in a solid state. Further, even when the solid-liquid phase change material is in a liquid phase, the outer shape of the body temperature regulation pack does not deform regardless of the orientation of the body temperature regulation pack. The body temperature regulation pack has a substantially plate shape as a whole, and has high heat absorbing ability or heat releasing ability. In a body temperature regulation pack (1) having the solid-liquidphase change material filled therein, the body temperature regulation pack (1) includes a plurality of bag members (T) arranged in parallel to each other. The plurality of bag members (T) are each filled with the solid-liquid phase change material without a gap.

[FIG. 1]

T
T
1
T
40
T
42
43a
43b

EP 2 657 629 A1

## Description

### Technical Field

The present invention relates to a body temperature regulation pack to be worn for preventing heat stress under a high temperature environment or for keeping the body warm under a cold environment, and more particularly, to a body temperature regulation pack using a solid-liquid phase change material, and a pack holder to which the body temperature regulation pack is attachable.

### Background Art

During work under a high temperature environment (under a hot environment, etc. in summer, etc.) or when protective clothing is worn, the problemof heat stress (heat stroke caused by accumulation of metabolic heat in the human body) tends to arise due to body temperature increase. Particularly, these days when the heat island phenomenon is remarkable, this risk is further increased.

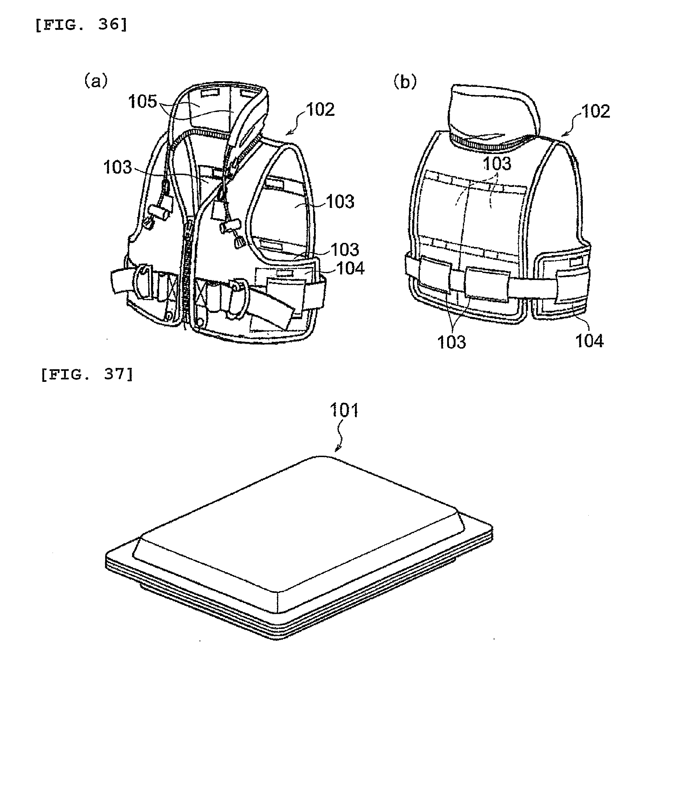

In view of the problem of heat stress as described above, conventionally, there has been proposed a cooling pack which is attached to the inner side of clothing and contains cooling material for preventing body temperature increase (see Patent Documents 1 and 2). For example, as illustrated in FIGS. 36 to 38, there has been proposed a square tabular cooling pack 101 which can be removably received in inner pockets 103, 104, and 105 of the clothing. By wearing the cooling pack 101, heat released from the body of a wearer is absorbed by the cooling material in the cooling pack 101 to prevent body temperature increase. As the cooling material, solid-liquid phase change materials are used, which change their physical phase fromsolidtoliquid by absorbing heat. Thesolid-liquid phase change materials are solidified at normal temperature.

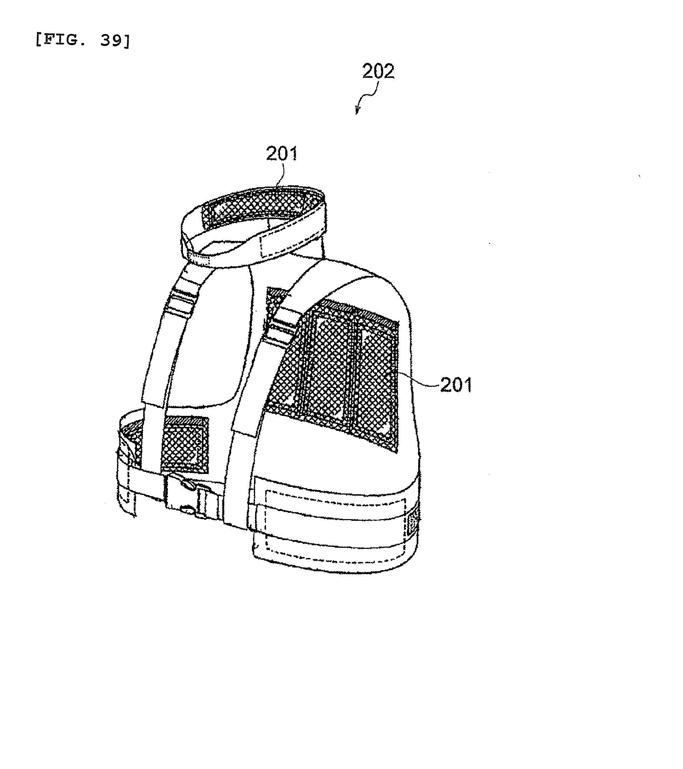

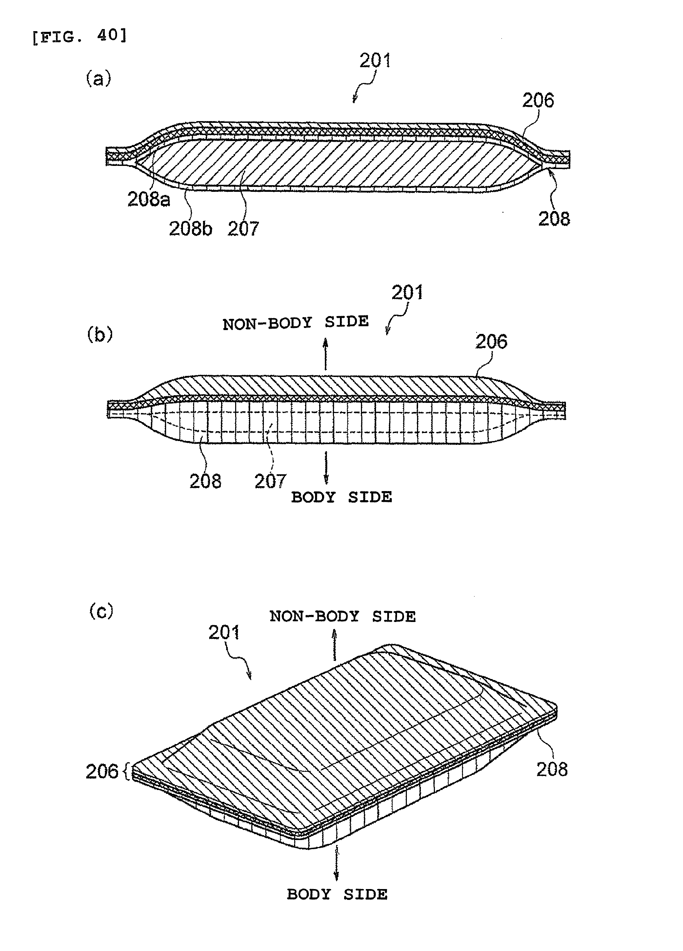

Further, as illustrated in FIG. 40, there has been proposed a cooling member 201 having a rectangular and substantially planar shape, which is filled with a gel refrigerant 207 which is always flexible and does not solidify even after cooling. The cooling member 201 is used by attached to the inner side of clothing 202 as illustrated in FIG. 39, and is cooled in a freezer before use.

### Citation List

### Patent Document

[Patent Document 1] Japanese Patent Laid-open No. 2003-328213
[Patent Document 2] Japanese Patent Laid-open No. 2010-255152

### Summary of Invention

### Technical Problems

However, when the flat tabular cooling pack 101 as proposed in Patent Document 1 is used, the cooling pack 101 is solidified into a solid form at normal temperature, and hence cannot be flexibly folded along the body. As a result, a gap may be generated between the body and the cooling pack 101. Therefore, the degree of contact between the cooling pack 101 and the body may be reduced, which may prevent the heat released from the body from being efficiently absorbed, reducing the cooling efficiency.

Further, for example, when clothing 102 is kept on a hanger while the cooling packs 101 are kept in the pockets 103, 104, and 105 when the cooling material was liquefied, as indicated by the dotted lines inFIG. 41, the liquefiedcoolingmaterial collects excessively at the lower portion of the pack 101 due to its own weight to deform the pack into an expanded shape. If the cooling material is cooled and solidified while maintaining this deformed state, a position where the cooling material is thick and a position where the cooling material is thin are generated in the cooling pack 101, and hence unevenness in cooling efficiency may be generated when the cooling pack 101 is reused. In order to prevent this unevenness, when the cooling material is cooled to return to a solid form, it is always necessary to take the cooling packs 101 out from the pockets 103, 104, and 105 of the clothing 102 and lay them out, which takes a time and effort. Further, when the cooling packs 101 are stored while in the clothing 102, it is necessary to lay the entire clothing 102 out to prevent deformation, which raises another problem in that a large space is required for the storage area.

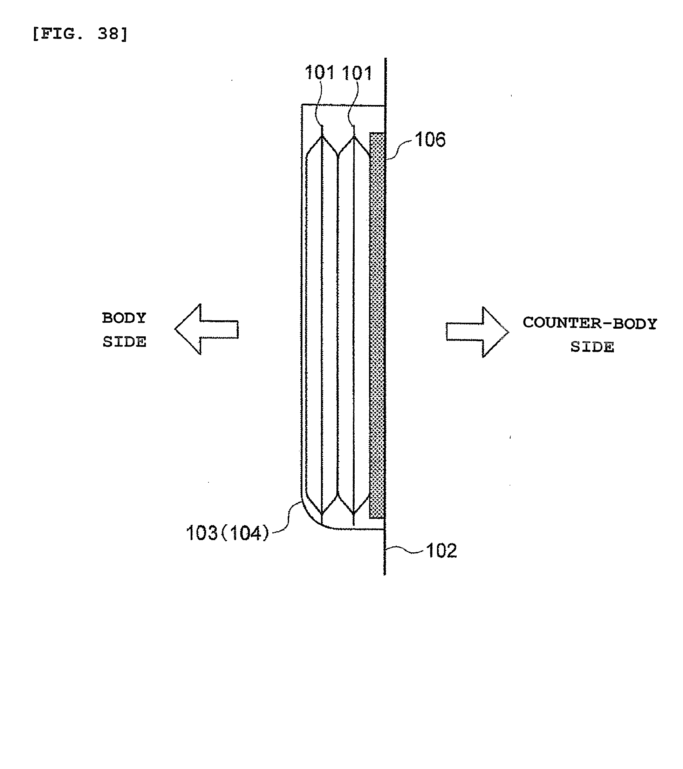

Further, when the cooling packs 101 are worn, a larger amount of cooling material is required to be arranged at the chest, back, etc., where a large amount of heat is released, while no cooling material or only a small amount of cooling material is required for a region where little heat is released, such as the abdomen. However, in the case of the tabular cooling pack 101, when a plurality of cooling packs 101 are used in an overlapping manner on the inner side of a heat insulatingpad 106 to increase the amount as illustrated in FIG. 38, heat conductivity is degraded, and an effect proportional to the increased amount cannot be expected. On the other hand, when the thickness is increased by a single cooling pack 101, the deformation when the cooling material is in a liquid phase becomes larger.

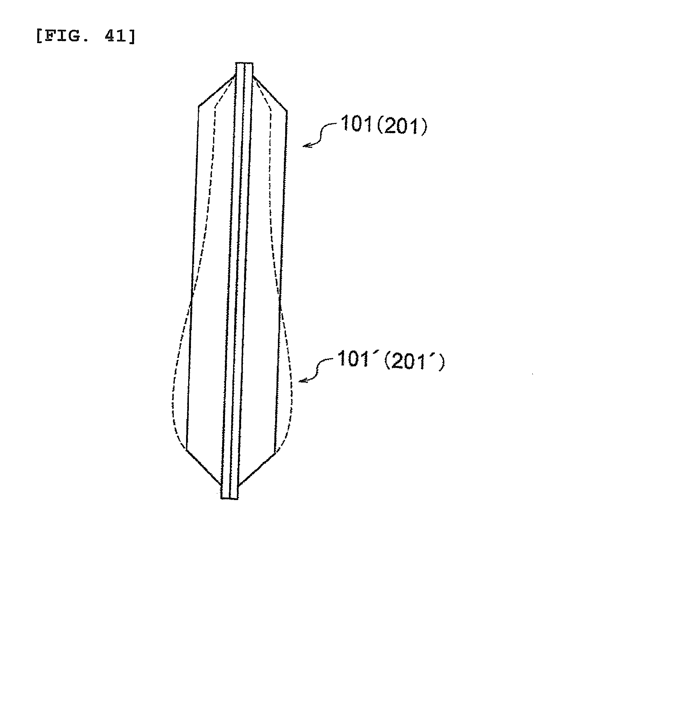

On the other hand, in the cooling member 201 as proposed in Patent Document 2, a rectangular and substantially planar pack 208 is filled with a gel refrigerant 207, and hence the cooling member 201 is flexible and easily comes into close contact with the body. However, similarly to the cooling pack of Patent Document 1, the refrigerant 207 easily collects at the lower portion of the pack 208 by its own weight to still deform as indicated by the dotted lines in FIG. 41. Therefore, even when it is desired to increase the amount of the refrigerant 207, the thickness of the cooling member 201 cannot be increased. Further, the refrigerant 207 is formed of 40% of a heat storage material (specific heat: 35 cal/g) which changes its phase, and the remaining 60% of water (specific heat: 1 cal/g). Therefore, as compared to a solid-liquid phase change material formed of a 100% heat storage material, the heat absorbing ability is low, and a low temperature lasting effect cannot be expected for a long period of time.

Further, when the solid- liquid phase change material is used as a heat retaining material for the body in a cold environment (such as in an environment where the cold is felt, in winter, etc.) in contrast to the above, the heat retaining pack is brought into close contact with the body in a state in which the solid- liquid phase change material is in a liquid phase. Thus, the heat retaining pack releases heat to exhibit the heat retaining effect, and then changes into a solid phase. Therefore, when the heat retaining pack is deformed as illustrated in FIG. 41 in the liquid phase state, unevenness is generated in the heat retaining effect. Further, in the case of the tabular heat retaining pack as illustrated in FIGS. 37 or 40, if the tabular heat retaining pack is wrapped all the way around the leg or the like when the solid- liquid phase change material is in the liquid phase and the solid- liquid phase change material changes into the solid phase under this state, the removal thereof becomes difficult.

The present invention has been made in view of the above-mentioned problems, and has a main object of providing a body temperature regulation pack as follows. The body temperature regulation pack uses a solid-liquid phase change material to prevent body temperature increases or body temperature decreases of the wearer. When the body temperature regulation pack is arranged on the inner side of a pack holder to be worn under clothing, the body temperature regulation pack may be folded along the body shape even in a solid state. Further, even when the solid-liquid phase change material used as a cooling material or a heat retaining material is in a liquid phase, the outer shape of the body temperature regulation pack does not deform regardless of the orientation of the body temperature regulation pack. The body temperature regulation pack has a substantially tabular as a whole, and has high heat absorbing ability or heat releasing ability.

Solution to Problems

According to the present invention, there is provided a body temperature regulation pack having a solid-liquid phase change material filled therein, the body temperature regulation pack including a plurality of bag members, in which the plurality of bag members are each filled with the solid-liquid phase change material without gaps.

It is preferred that the plurality of bag members each have an elongated cylindrical shape. Further, the body temperature regulation pack of the present invention may be formed by bonding together surrounding edge portions of two inner sheets, and the elongated bag-like portions may be separated from each other by welding a plurality of positions of the two inner sheets in parallel to each other. Still further, one surface of the body temperature regulation pack of the present invention may be covered with a heat insulating material.

The solid-liquid phase change material used in the body temperature regulation pack of the present invention may contain sodium acetate, sodium sulfate, or normal paraffin.

According to another aspect of the present invention, there is provided a torso pack holder for holding the body temperature regulation pack onto a body side, the torso pack holder being configured to enable removable attaching of the body temperature regulation pack at any position.

The torso pack holder includes a foreside portion for covering the chest of a wearer, and a backside portion for covering the back of the wearer, and the foreside portion and the backside portion may be disengageably connected to each other. Further, the foreside portion and the backside portion may be connected to each other with a stretchable band-like member.

According to still another aspect of the present invention, there is provided a collar pack holder and a leg pack holder for removably wearing the body temperature regulation pack around the neck and legs, respectively.

Advantageous Effects of the Invention

When the body temperature regulation pack of the present invention is used for the purpose of cooling the body, a solid-liquid phase change material having a high heat absorbing ability is used. The body temperature regulation pack folds along the shape of the body even in the solid phase, and hence the degree of body contact and cooling efficiency are high. Further, the body temperature regulation pack does not deform even when the amount of the solid-liquid phase change material per unit area is increased, and hence the amount of the solid-liquid phase change material can be appropriately adjusted according to the body region. Therefore, heat stress in high temperature environments can be efficiently reduced.

Further, the solid- liquidphase change material is used as the cooling material, and hence even when the solid-liquid phase change material changes into a liquid phase and loses its cooling function, the solid- liquid phase change material can be cooled to return to the solidphase again. Thus, the solid- liquidphase change material can be repeatedly used over and over again. Therefore, the solid- liquid phase change material is more economical compared to a disposable cooling material.

Further, as the body temperature regulation pack can be removably attached in any position of the pack holder, the heat can be absorbed at necessary positions, and in addition, heat release positions can be formed as well.

Further, at the same time, when the body temperature regulation pack of the present invention is used for the purpose of keeping the body warm, the body temperature regulation pack itself does not deform even in the liquid phase state, and hence the body can be kept warm at necessary positions without unevenness. Further, even when the solid-liquid phase change material is in the solid phase, the body temperature regulation pack can be freely folded or extended, and hence the body temperature regulation pack folded along the shape of the body can be easily removed. Therefore, during outdoor work in the winter or in a work place without a heating device, not only the upper body but also the feet of the wearer can be kept warm by wrapping the body temperature regulation pack on the calf or the like on the outside of the clothing.

Brief Description of the Drawings

[FIG. 1] Perspective view of a body temperature regulation pack of the present invention.
[FIG. 2] Perspective view of a cross section of the body temperature regulation pack illustrated in FIG. 1 according to a first embodiment of the present invention.
[FIG. 3] Schematic view illustrating a step of manufacturing the body temperature regulation pack illustrated in FIG. 2, which represents a state in which a pair of inner sheets are overlapped with each other to form an inner pack.
[FIG. 4] Schematic view illustrating a step of manufacturing the body temperature regulation pack illustrated in FIG. 2, which represents a state in which a solid-liquid phase change material is injected into elongated bag-like portions of the inner pack.
[FIG. 5] Schematic view illustrating a step of manufacturing the body temperature regulation pack illustrated in FIG. 2, which represents a state in which the injected solid-liquid phase change material changes into a solid phase, and then is subjected to vacuum packing.
[FIG. 6] Schematic view illustrating a step of manufacturing the body temperature regulation pack illustrated in FIG. 2, which represents a state in which the solid-liquid phase change material is made to return to a liquid phase again, and then an upper edge portion of the pack is folded.
[FIG. 7] Schematic view illustrating a step of manufacturing the body temperature regulation pack illustrated in FIG. 2, which represents a state in which all of the surrounding edge portions are folded, and the inner pack is filled with the solid-liquid phase change material without a gap.
[FIG. 8] Schematic view illustrating a step of manufacturing the body temperature regulation pack illustrated in FIG. 2, which represents a state in which a heat insulating material is fixed to the inner pack.
[FIG. 9] Schematic view illustrating a step of manufacturing the body temperature regulation pack illustrated in FIG. 2, which represents a state in which the inner pack having the heat insulating material fixed thereon is covered with an outer pack.
[FIG. 10] Schematic view illustrating a step of manufacturing the body temperature regulation pack illustrated in FIG. 2, which represents a state in which, after the inner pack is covered with the outer pack, vacuum packing is performed to complete the body temperature regulation pack.
[FIG. 11] Schematic view illustrating flexibility of the completed body temperature regulation pack illustrated in FIG. 2.
[FIG. 12] Schematic view illustrating a state in which a plurality of body temperature regulation packs illustrated in FIG. 2 are stored in an overlapping manner.
[FIG. 13] Schematic view illustrating a step of manufacturing an elongated bag member according to a second embodiment of the present invention, which represents a state in which one end portion of the pack is welded.
[FIG. 14] Schematic view illustrating a step of manufacturing the elongated bag member according to the second embodiment of the present invention, which represents a state in which a solid-liquid phase change material is filled into the interior thereof, and then an end portion that has been an injection port is welded.

[FIG. 15] Schematic view illustrating a step of manufacturing the elongated bag member according to the second embodiment of the present invention, which represents a state in which the welded portions at both ends are inwardly folded.

[FIG. 16] Schematic view illustrating a step of manufacturing the body temperature regulation pack illustrated in FIG. 1 by employing the second embodiment, which represents a state in which the elongated bag members illustrated in FIG. 15 are arranged on an elongated bag member arraying member so that the elongated bag members are arrayed in parallel to each other in a parallel direction.

[FIG. 17] Perspective view illustrating a step of manufacturing the body temperature regulation pack illustrated in FIG. 1 by employing the second embodiment, which represents the elongated bag member arraying member illustrated in FIG. 16.

[FIG. 18] Schematic view illustrating a step of manufacturing the body temperature regulation pack illustrated in FIG. 1 by employing the second embodiment, which represents a state in which a heat insulating material is fixed to the plurality of elongated bag members illustrated in FIG. 16.

[FIG. 19] Schematic view illustrating a step of manufacturing the body temperature regulation pack illustrated in FIG. 1 by employing the second embodiment, which represents a state in which a group of the elongated bag members having the heat insulating material fixed thereon is covered with an outer pack.

[FIG. 20] Schematic view illustrating a step of manufacturing the body temperature regulation pack illustrated in FIG. 1 by employing the second embodiment, which represents a state in which, after the group of the elongated bag members is covered with the outer pack, vacuum packing is perform to complete the body temperature regulation pack.

[FIG. 21] Schematic view illustrating a step of manufacturing the body temperature regulation pack illustrated in FIG. 1 by employing the second embodiment, which represents a state in which the elongated bag member arraying member and a heat insulating material according to another embodiment of the present invention are fixed to the plurality of elongated bag members illustrated in FIG. 16.

[FIG. 22] View illustrating a state in which the amount of the solid-liquid phase change material to fill the body temperature regulation pack of the present invention is changed as appropriate. The width is set to 20 cm, and the diameters of the elongated bag-like portions are set to 2 cm, 2.5 cm, 3 cm, 4 cm, 4.5 cm, and 5 cm from the top.

[FIG. 23] Schematic view illustrating a cooling pedestal to be used in a case where the body temperature regulation pack illustrated in FIG. 1 is used as a cooling material, and when the body temperature regulation pack is cooled in a refrigerator.

[FIG. 24] Schematic view illustrating a state in which the body temperature regulation pack illustrated in FIG. 1 is cooled with use of the cooling pedestal illustrated in FIG. 23.

[FIG. 25] Schematic view of a body temperature regulation pack to be worn around the neck.

[FIG. 26] Perspective view illustrating a mesh bag made of a mesh material into which the body temperature regulation pack illustrated in FIG. 25 is inserted.

[FIGS. 27] Plan views illustrating a front surface and a rear surface of the mesh bag made of the mesh material illustrated in FIG. 26.

[FIG. 28] Front view illustrating a pack holder made of a mesh material dedicated for holding the body temperature regulation pack according to an embodiment of this application.

[FIG. 29] Rear view of the pack holder illustrated in FIG. 28.

[FIG. 30] Side view of the pack holder illustrated in FIG. 28.

[FIGS. 31] Views illustrating a front surface and a rear surface of a foreside portion of the pack holder illustrated in FIG. 28.

[FIG. 32] View illustrating a front surface and a rear surface of a backside portion of the pack holder illustrated in FIG. 28.

[FIG. 33] View illustrating a state in which the foreside portion illustrated in FIGS. 31 and the backside portion illustrated in FIG. 32 are connected to each other by band-like members.

[FIGS. 34] Views illustrating a front surface, a rear surface, and a modified example of a collar portion of the pack holder illustrated in FIG. 28.

[FIG. 35] View illustrating a leg pack holder.

[FIGS. 36] Perspective views illustrating a conventional vest for holding a cooling pack.

[FIG. 37] Perspective view illustrating a conventional cooling pack illustrating in FIGS. 36.

[FIG. 38] Sectional view illustrating a state in which the conventional cooling packs illustrated in FIGS. 36 are used in an overlapping manner.

[FIG. 39] Perspective view illustrating a conventional vest for holding a cooling pack.

[FIGS. 40] Perspective views illustrating a conventional cooling pack illustrated in FIG. 39.

[FIG. 41] Elevational view illustrating the conventional cooling pack and its deformation.

Description of Embodiments

In the following, preferred embodiments of the present invention are described with reference to the drawings.

[Body Temperature Regulation Pack]

(First Embodiment)

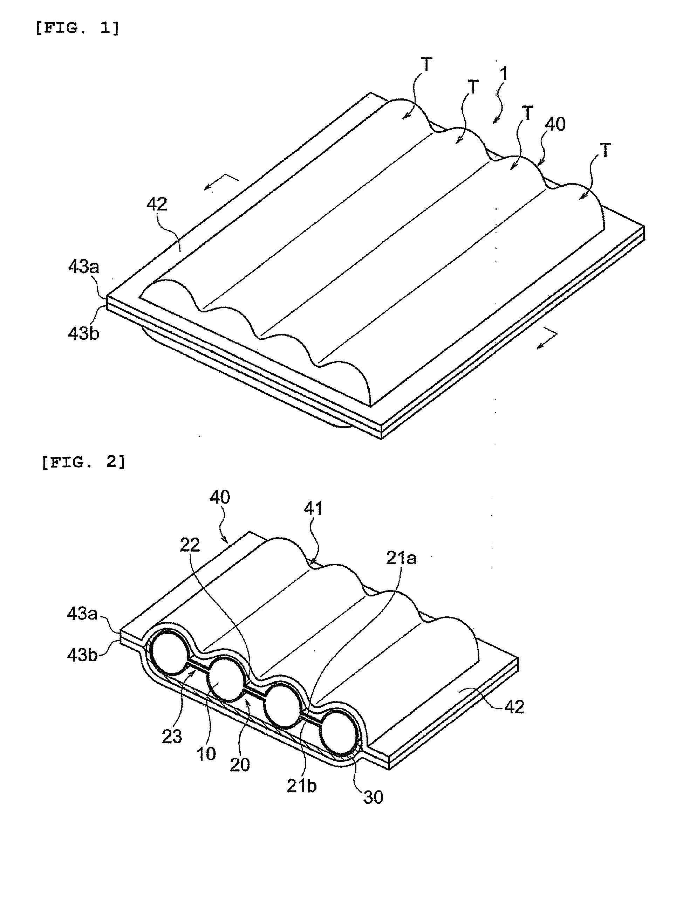

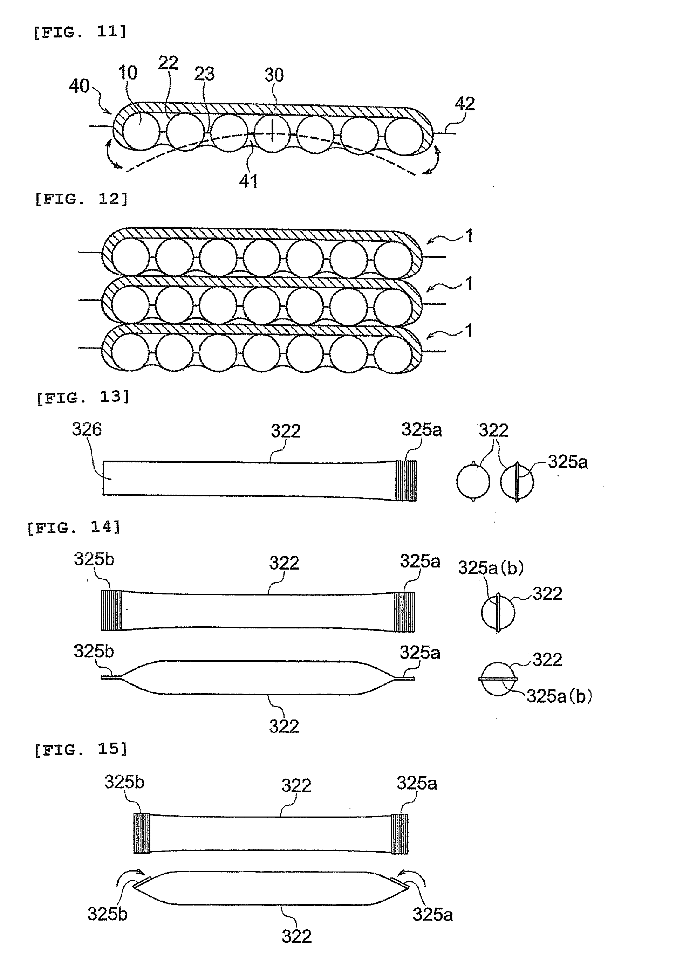

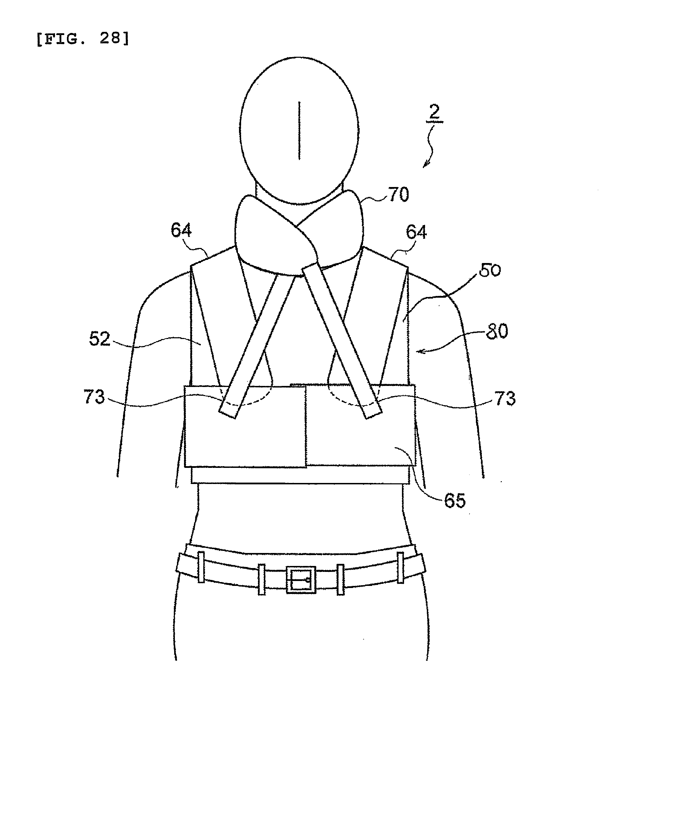

First, description is made of a body temperature regulation pack 1 which is used by being attached to a pack holder 2 (see FIG. 28) to be described later.
As illustrated in FIG. 1, the entire body temperature regulation pack 1 is formed into a substantially tabular rectangular shape, and includes a plurality of bag members T arranged in parallel to each other. As illustrated in FIG. 2, in a first embodiment of the present invention, the interior of the body temperature regulation pack 1 is divided into a plurality of elongated bag- like portions 22, and the body temperature regulation pack 1 can be flexibly folded due to welded portions 23 to be described later, which are provided between the respective elongated bag- like portions 22 and serve as joints. Note that, the shape of the body temperature regulation pack 1 is not limited to a rectangular shape as in the embodiment of this application, and may be another shape.
The body temperature regulation pack 1 includes the elongated bag-like portions 22 each filled with a solid-liquid phase change material 10, an inner pack 20 including the plurality of elongated bag-like portions 22, a heat insulating material 30 for covering one surface of the inner pack 20, and an outer pack 40 for covering the entire inner pack 20 having the heat insulating material 30 fixed thereon. The surface of the body temperature regulation pack 1 has a concavo-convex shape along the shape of the elongated bag-like portions 22 inside the outer pack 40.
The inner pack 20 includes a pair of inner sheets 21a and 21b. In this embodiment, two inner sheets 21a and 21b having the same shape and made of the same material are used, but a single sheet may be folded to form a pair. The two inner sheets 21a and 21b overlaps with each other, and surrounding edge portions 25 thereof are bonded together by welding or the like, to thereby form the inner pack 20. Further, between a pair of bonded edge portions 25 on two sides, the plurality of elongated welded portions 23 are provided in parallel to each other, to thereby form the elongated bag-like portion 22 between the respective welded portions 23. In the present invention, the inner sheets 21a and 21b are welded in the parallel direction at regular intervals, but the present invention is not limited thereto. By changing the welding intervals as appropriate, the size of each elongated bag-like portion 22 can be changed.
The inner sheets 21a and 21b each have a three-layer structure in which a sheet-like polyethylene, aluminum foil, and a sheet-like nylon (trademark) are bonded together. Those three layers are arranged so that, when the inner sheets 21a and 21b are used for the inner pack 20, polyethylene, the aluminum foil, and nylon (trademark) are stacked in this order from the inner side of the inner pack 20 to the outer side of the inner pack 20. With this structure, the inner sheets 21a and 21b have flexibility and high strength, and hence are not easily damaged. Further, the aluminum foil is used for the inner sheets 21a and 21b, and hence the water vapor barrier property is improved. Thus, moisture is prevented from being evaporated from the interior of the inner pack 20, and thus the function of the solid-liquid phase change material 10 is prevented from being lost. Further, with use of the aluminum foil, the heat conductivity is improved to increase the cooling efficiency. Further, nylon (trademark) isusedfortheoutersurface of each of the inner sheets 21a and 21b, and thus the resistance to abrasion is improved. Note that, as long as the inner sheets 21a and 21b are flexible and have sufficient strength and the solid-liquid phase change material 10 sealed therein does not leak out, the materials are not particularly limited to those in this embodiment.
Inside the elongated bag-like portion 22, the solid-liquid phase change material 10 is filled in the elongated bag-like portion to the capacity without a gap, to thereby form an elongated columnar shape. Therefore, even when the body temperature regulation pack 1 is arranged in a vertical direction in a state in which the solid-liquid phase change material 10 is in a liquid phase, there is no margin for the elongated bag-like portion 22 to generate a deflection, and hence the elongated bag-like portion 22 is not deformed. Further, the plurality of elongated bag-like portions 22 are arrayed in the parallel direction through the intermediation of the flexible welded portions 23, and hence even when the solid-liquid phase change material 10 is in a solid phase, as illustrated in FIG. 11, the body temperature regulation pack 1 can be folded along the shape of the body due to the welded portions 23 serving as joints. Thus, the degree of contact of the body temperature regulation pack 1 with the human body can be increased. Further, the column-shaped elongated bag-like portions 22 are arrayed in parallel to each other in the lateral direction, and hence a substantially tabular shape having a surface with a concavo-convex shape is obtained as a whole. Therefore, when the plurality of body temperature regulation packs 1 are stored, as illustrated in FIG. 12, the body temperature regulation packs 1 can be stably stored in an overlapping manner, and no excess space is required. Note that, if a conventional flat tabular body temperature regulation pack as illustrated in FIGS. 37 and 40 is filled with the cooling material without a gap to prevent deformation even in the liquid phase, the entire pack takes on a shape in which its center part is expanded into a rugby ball shape when viewed from the lateral side. Thus, the body temperature regulation packs cannot be stably stored in an overlapping manner, and the degree of contact to the body becomes very low in the solid phase.

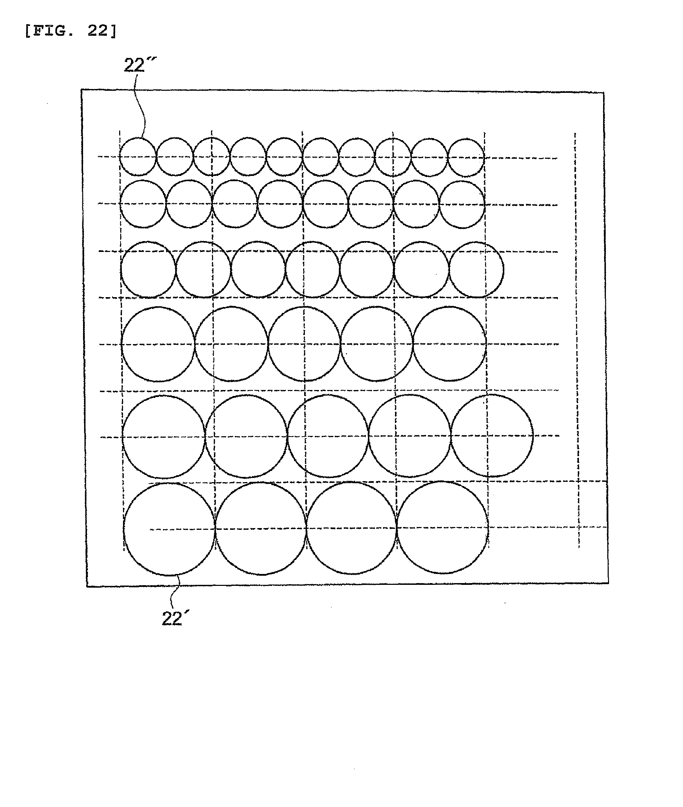

In the temperature regulation pack 1, there is no need to worry about deformation even when the amount of the solid-liquid phase change material 10 is increased. Therefore, as illustrated in FIG. 22, by changing the diameter of the elongated bag-like portion 22 as appropriate, the amount of the solid-liquid phase change material 10 can be adjusted in accordance with the body region and the necessary cooling function retention time or temperature retaining function retention time. Specifically, when the solid-liquid phase change material 10 is used as the coolingmaterial, the following adjustment is made. A larger amount of the solid-liquid phase change material 10 is arranged at the chest, back, etc., where a large amount of heat is released (by increasing the diameter of a cylindrical elongated bag-like portion 22'/lower side of FIG. 22), and a smaller amount of the solid-liquid phase change material is arranged at a region in which little heat is released (by decreasing the diameter of a cylindrical elongated bag-like portion 22''/upper side of FIG. 22). Further, when a part of the circular section of each of the elongated bag-like portions 22 is brought into contact with the body, heat released from the body surface can be efficiently absorbed, and abnormal increases in body temperature can be prevented. Further, in contrast, when the solid-liquid phase change material 10 is used as the heat retaining material, the solid-liquid phase change material 10 releases heat to be transferred to the body, which enables efficient warming.

The solid-liquid phase change material 10 can first be used as a cooling material. When the solid-liquid phase change material changes from the solid phase state to the liquid state, heat energy called latent heat is necessary, which is absorbed without changing the temperature in order to change the substance binding state. That is, the solid-liquid phase change material 10 absorbs the heat energy released from the body of the wearer, and during the change from the solid state to the liquid state, the temperature of the solid-liquid phase change material 10 is fixed to a constant temperature called a melting point. Thus, an excess increase of the body temperature of the wearer is prevented. The melting point of the solid-liquid phase change material 10 is preferably a temperature lower than a general human body surface temperature (32°C) at rest. In this embodiment, sodium sulfate decahydrate is used as a base material of the solid-liquid phase change material 10. The employed sodium sulfate decahydrate contains an additive such as a super-cooling prevention agent in order to maintain a stable reversibility as a hydrate. The sodium sulfate decahydrate itself has a melting point of a little more than 32°C. As a result of super-cooling, the solid-liquid phase change material 10 undergoes partial solid-liquid conversion to change its phase in a range of 25°C to 28°C, which is lower than 32°C. Therefore, as compared to a cooling material which uses a conventional solid-liquid phase change material having a melting point around 0°C, there is no fear of frostbite due to excessive cooling of the body, or reduction of the body function. Further, when the phase change temperature is in the range of 25°C to 28°C, the wearer barely feels cold at rest (when no heat adsorption is required) even when the body temperature regulation pack 1 is pressed from the top of the underwear or the work clothes, and no phase change occurs in the solid-liquid phase change material 10. However, when stress is applied to the body and the body surface temperature is increased, coolness is suddenly felt, and the phase change of the solid-liquid phase change material 10 starts. On the other hand, in a cooling material using a conventional solid-liquid phase change material which changes its phase around 0°C, the solid-liquid phase change occurs at normal body temperature. Therefore, at the time point when the prevention of abnormal body temperature increase becomes necessary, the solid-liquid phase change material is already liquefied and its cooling function is lost. This is a significant difference with respect to the conventional cooling material which changes its phase around 0°C.

Further, even when the body temperature regulation pack 1 is used in a high temperature environment and the solid- liquid phase change material 10 which has absorbed heat is in the liquid phase, the phase can return to a solid state in a relatively short period of time. For example, when the body temperature regulation pack 1 is cooled by immersing the body temperature regulation pack 1 in ice water for a few minutes, the heat is easily released, and the phase can return to a solid state without using facilities such as a refrigerator or a cooling box. In general, in a case of the elongated bag- like portion 22 having a diameter of more than 40 mm, the phase can return to a solid state in one night at a temperature of around 10°C. Further, even when the solid- liquid phase change material 10 turns completely into a liquidphase and loses its cooling function, the solid- liquid phase change material 10 may be cooled to release its heat so that the phase returns to a solid phase again. Thus, the solid- liquid phase change material 10 can be used over and over again. Therefore, the solid- liquid phase change material is more economical than a disposable cooling material. Note that, the base material of the solid- liquid phase change material 10 is not limited to the sodium sulfate decahydrate of this embodiment as long as the material undergoes a 100% solid- liquid phase change, and the phase change temperature is in the range of room temperature (about 15°C to 35°C) . Other inorganic salt hydrate based substances (such as sodium phosphate dodecahydrate) or normal paraffin (such as n- heptadecane, n- octadecane, and n- nonadecane) may be used.

The cooling material 10 of the invention of this application contains a 100% solid-liquid phase change material. On the other hand, the cooling material 207 of the cooling member 201 illustrated in FIGS. 40 corresponds to contents having main components of 40% of the phase change material and the remaining percentage of water having a specific heat of 1 or lower. Therefore, even when the cooling material 207 is cooled, the cooling material 207 does not turn into a solid phase and always has flexibility. However, when the heat absorption ability of 1 pack of 100 g is calculated simply, presuming that the heat absorption at the time of phase change of the heat storing agent which changes its phase at 25 to 30°C is 35 cal/g, in a case where the temperature is increased from 25°C to 30°C, the heat absorption amount of the cooling member 201 illustrated in FIGS. 40, which contains 40% of the solid-liquid phase change material and 60% of water having a specific heat of 1, is as follows:

$$40\ g \times 35\ cal + 60\ g \times 1\ cal \times 5°C = 1{,}700\ cal.$$

In contrast, the heat absorption amount of the body temperature regulation pack 1 of the present invention, which contains a 100% solid-liquid phase change material, is as follows:

$$100\ g \times 35\ cal = 3{,}500\ cal.$$

Therefore, the body temperature regulation pack 1 of the present invention and the conventional cooling member illustrated in FIGS. 40 have a difference in heat absorption ability of about two times, and when the pack is used by being worn in a vest-shaped pack holder 2 to be described later, there is a crucial difference in the low temperature lasting effect. In this case, the body temperature regulation pack 1 to be used in the present invention can be freely folded along the shape of the body as described above. Therefore, the body temperature regulation pack 1 satisfies both the high heat absorption ability, which is obtained by having the 100% solid-liquid phase change material in the cooling material 10, and properties for following the human body.

The solid-liquid phase change material 10 is crystallized in the solid phase, and when this crystal is completely melted, it becomes difficult to return the phase to the liquid phase. Specifically, for example, when the body temperature regulation pack 1 is heated by 100°C hot water, the crystal is completely eliminated in a few minutes. Under this state, when the body temperature regulation pack 1 is left at normal temperature (about 22°C) to wait for the solid-liquid phase change, it takes a long period of time, and in addition, the phase may not return to the solid phase. In this case, the solid-liquid phase change material 10 is once cooled to about 10°C so that crystals even in a small amount are formed therein. After that, the phase can return to the solid phase at normal temperature. That is, in other words, by causing the crystal to remain in the body temperature regulation pack 1 even in a small amount, it becomes possible to reduce the energy to be spent for changing the phase from the liquid phase to the solid phase. Therefore, when the body temperature regulation pack 1 is adjusted with respect to the body, it is necessary to design the diameter so as to be slightly larger than the necessary size, to thereby prevent complete crystal dissolution.

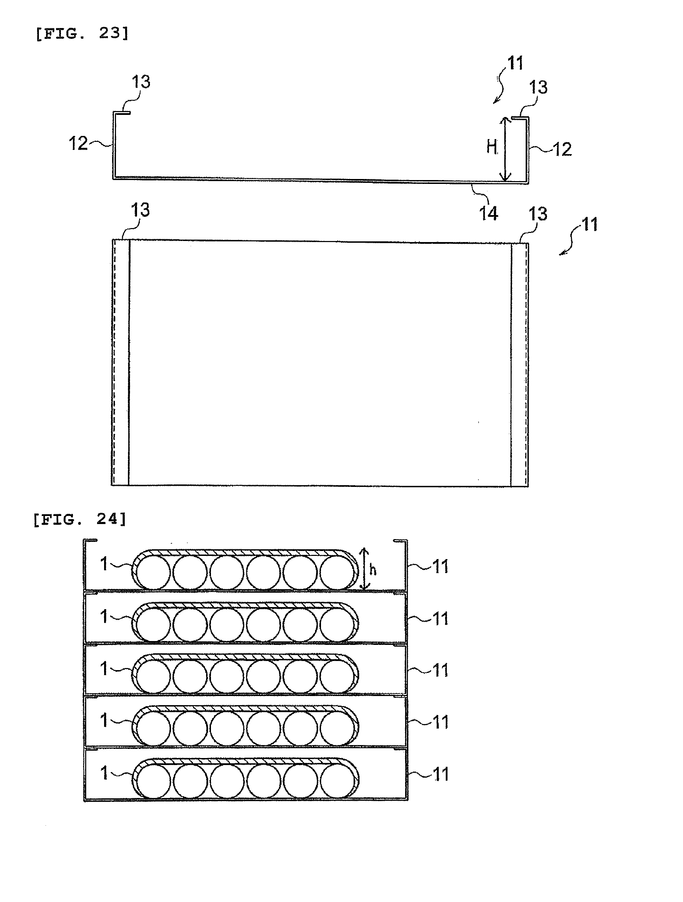

In this case, the body temperature regulation pack 1 having a large-diameter elongated bag-like portion 22' as illustrated in FIG. 22 requires a longer period of time to change from the liquid phase to the solid phase as compared to the body temperature regulation pack 1 having a small-diameter elongated bag-like portion 22''. Therefore, in order to increase the working efficiency, when the function of the body temperature regulation pack 1 having the large-diameter elongated bag-like portion 22' needs to be recovered efficiently, a method of cooling the body temperature regulation pack 1 in a refrigerator is recommended more than releasing the heat at normal temperature. At this time, when the plurality of body temperature regulation packs 1 are cooled in a refrigerator, the cooling pedestal 11 illustrated in FIGS. 23 and 24 is used.

As illustrated in FIG. 23, the cooling pedestal 11 has a substantially rectangular shape when viewed from above and includes erected portions 12 on two opposing short sides. Further, as illustrated in FIG. 24, along an upper end of each of the erected portions 12, a locking portion 13 is provided inwardly in the cooling pedestal 11 so as to be perpendicular to the erected portion 12. The height H of the erected portion 12 is set to be larger than the thickness h of the body temperature regulation pack 1. Note that, the cooling pedestal 11 is formed of aluminum plate. Also, note that, the shape of the cooling pedestal 11 is not limited to that of this embodiment as long as the shape allows passage and easy circulation of cold air, and the erected portions 12 may be provided on two opposing long sides. Further, the material of the cooling pedestal 11 is not limited to aluminum plate, and is only required to be a material having high heat conductivity.

How to use the cooling pedestal 11 is now described. As illustrated in FIG. 24, a single body temperature regulation pack 1 is placed on each cooling pedestal 11. At this time, the side on which the heat insulating material 30 is present is directed upward. By directing the side on which the heat insulating material 30 is not arranged downward so as to obtain close contact with the cooled aluminum plate, the body temperature regulation pack 1 can be efficiently cooled. Next, the cooling pedestals 11 each having a body temperature regulation pack 1 placed thereon are stacked as a rack. The bottom surface 14 of the cooling pedestal 11 to be stacked engages with the locking portion 13 of the underlying cooling pedestal 11. Thus, stable stacking is possible. Note that, the number of the body temperature regulation packs to be placed on the cooling pedestal 11 is not limited to one, and as long as the body temperature regulation packs 1 do not overlap each other and all of the body temperature regulation packs 1 are brought into close contact with the aluminumplate, a plurality of body temperature regulation packs may be placed on a single cooling pedestal 11.

As described above, the cooling pedestals 11 are used to stack the body temperature regulation packs 1, and cooling is performed in a refrigerator. Thus, no extra space is required. Further, the height H of the erected portion 12 is larger than the thickness h of the body temperature regulation pack 1, and thus an interval is provided between the body temperature regulation pack 1 and the cooling pedestal 11 stacked thereabove. In this manner, cold air passes through the interval and the body temperature regulation pack 1 can be efficiently cooled. Further, the cooling pedestal 11 is formed of aluminumplate having high heat conductivity, and hence the body temperature regulation pack 1 can be cooled more efficiently.

Further, the solid- liquid phase change material 10 can be used as a heat retainingmaterial as well. The reason is as follows. With use of the solid- liquid phase change material 10 made of a substance with high freezing- point temperature (for example, sodium acetate trihydrate), the body can be kept warm by the heat released when the phase changes from the liquid phase to the solid phase.

Note that, the base material of the solid- liquid phase change material 10 is not limited to the sodium acetate trihydrate of this embodiment, and it is only required that the base material be a material which undergoes a solid liquid phase change of nearly 100% and has a phase change temperature range that protects the human body from the surrounding cold environment to feel comfort (about 15°C to 50°C) . Other inorganic salt hydrate based substances (such as sodium sulfate decahydrate and sodium phosphate dodecahydrate) or normal paraffin (such as n- heptadecane, n- octadecane, and n- nonadecane) may be used.

With use of the solid-liquid phase change material 10 as the heat retaining material, during outdoor work in the cold months or in a work place without a heating device, the chilling of a worker can be relieved. In general, it is relatively easy for the worker to keep the upper body warm, but warming against the cold in the feet tends to be neglected. However, the body temperature regulation pack 1 of the present invention does not deform even in the liquid phase state, and hence necessary positions (leg parts or the like) of the body can be kept warm without unevenness. Further, even when the solid-liquid phase change material is in the solid phase, the body temperature regulation pack 1 can be freely folded or extended, and hence the body temperature regulation pack 1 folded along the shape of the body can be easily removed. Therefore, by wrapping the body temperature regulation pack 1 around the calf or the like from the top of the clothing such as pants, chilling in the feet can be easily relieved.

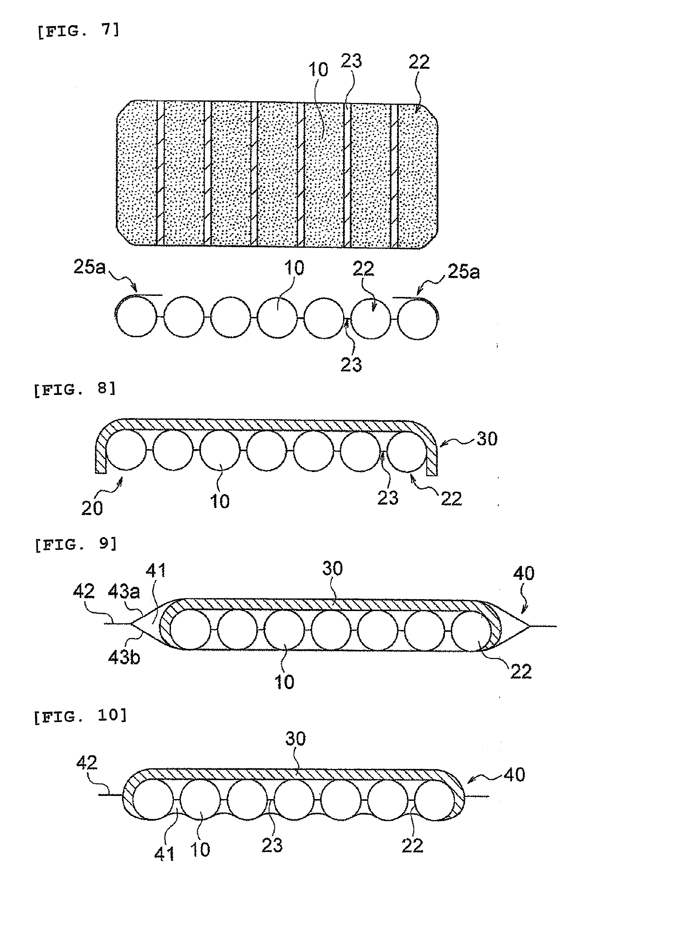

The heat insulating material 30 is a sheet-like foamed polyethylene in this embodiment. As illustrated in FIG. 2, the heat insulating material 30 is fixed so as to cover one surface and side portions of the inner pack 20 (see FIGS. 8 to 10). The "one surface" referred to herein corresponds to a surface on a side opposite to a surface at which the body temperature regulation pack 1 comes into contact with the body of the wearer. Further, the "side portion" corresponds to a side surface part extending from the one surface of the inner pack 20 to reach the surface on the opposite side. When the heat stress test is performed, the outer sides of the elongated bag-like portions 22 located at both ends of the inner pack 20 are liquefied first, and hence it is preferred to cause the heat insulating material 30 to wrap those side portions. The heat insulating material 30 has a substantially planar tabular shape with s sufficient area to cover the entire elongated bag-like portions 22 filled with the solid-liquid phase change material 10 inside the inner pack 20. In this manner, when the solid-liquidphase change material 10 is used as the cooling material, the heat released from the body is absorbed by the solid-liquid phase change material 10, while outside heat is kept from being transferred to the solid-liquid phase change material 10 by the heat insulating material 30. Thus, the cooling function of the solid-liquid phase change material 10 is less affected from the outside. Therefore, even under a high temperature environment, the cooling effect of the body temperature regulation pack 1 can be sufficiently exhibited, and the cooling effect is maintained for a long period of time. Further, when the solid-liquid phase change material 10 is used as the heat retaining material, the solid-liquid phase change material 10 is covered with the heat insulating material 30, and hence the heat released from the solid-liquid phase change material 10 does not escape to the outside. Therefore, the heat efficiently transfers to the body of the wearer for heat retention. In this case, the heat insulating material 30 is arranged inside the body temperature regulation pack 1, and hence the heat insulating material need not be provided in the pack holder 2 to be described later, and the weight of the pack holder 2 can be reduced. Note that, as long as the heat insulating material 30 is hard enough to prevent crushing when the inner pack 20 is sealed into the outer pack 40 and vacuum packing is performed, the material of the heat insulating material 30 is not limited to the foamed polyethylene of this embodiment and another substance having a sufficient heat insulating function may be used. Further, it is preferred that the heat insulating material 30 have a heat reflection property of reflecting heat rays including direct sunlight as much as possible. Further, the heat insulating material 30 preferably has a shock absorption property because it then becomes unnecessary for the pack holder 2 to absorb shock, but the pack holder 2 itself may have a shock absorption property.

The outer pack 40 is formed into a bag shape by bonding together a pair of outer sheets 43a and 43b, and includes an outer bag-like portion 41 having a bag shape with a hollow interior, and an ear-like portion 42 formed around the outer bag-like portion 41. Inside the outer bag-like portion 41, the inner pack 20 filled with the solid-liquid phase change material 10 and the heat insulating material 30 covering the one surface and the side portions of the inner pack 20 are sealed in vacuum. The outer pack 40 covers the inner pack 20 and the heat insulating material 30, to thereby protect the inner pack 20 from damage and the like, and to prevent dust or the like from accumulating at the welded portion 23 which is a portion connecting the elongated bag-like portions 22. Further, vacuum packing is performed when the outer pack 40 covers the inner pack 20 and the heat insulating material 30. Therefore, the outer pack 40 and the inner pack 20 are normally in a close contact state, but if by any chance a pinhole is opened from the outside, air enters the space between the outer pack 40 and the inner pack 20, and thus the tightness of the vacuum-packed outer pack 40 is released to generate sagging. Therefore, from this state of the outer pack 40, it can be easily confirmed somewhere that there is a pinhole. Further, this acts as a sensor where it can be presumed that a pinhole may be similarly opened in the inner pack 20.

The outer sheets 43a and 43b each have a three-layer structure in which a sheet-like polyethylene, aluminum foil, and a sheet-like nylon (trademark) are bonded together. These three layers are arranged so that, when the outer sheets 43a and 43b are used for the outer pack 40, polyethylene, the aluminum foil, and nylon (trademark) are stacked in this order from the inner side of the outer pack 40 to the outer side of the outer pack 40. With this structure, the outer sheets 43a and 43b have flexibility and high strength, and hence are not easily damaged. Further, the aluminum foil is used for the outer sheets 43a and 43b, and hence a water vapor barrier property is provided not only for the inner pack 20 but also for the outer pack 40. If by any chance the water vapor barrier of the inner pack 20 is damaged, due to the presence of this outer pack 40, the function of the solid-liquid phase change material 10 can be maintained as a whole. Further, polyethylene and nylon (trademark) are generally transparent. Therefore, by merely bonding the polyethylene and nylon (trademark) together, the inner pack and the heat insulating material can be seen from the outside, which is poor in appearance. The aluminum foil is used to function as a blind which prevents the interior of the outer pack 40 from being viewed. Further, the aluminum foil improves the heat conductivity to increase the cooling efficiency or the heat retaining efficiency. Further, by using nylon (trademark) in the outer surfaces of the outer sheets 43a and 43b, the resistance to abrasion is improved. Note that, as long as the outer sheets 43a and 43b are flexible and have sufficient strength and the internal vacuum state can be maintained, the materials are not particularly limited to those in this embodiment.

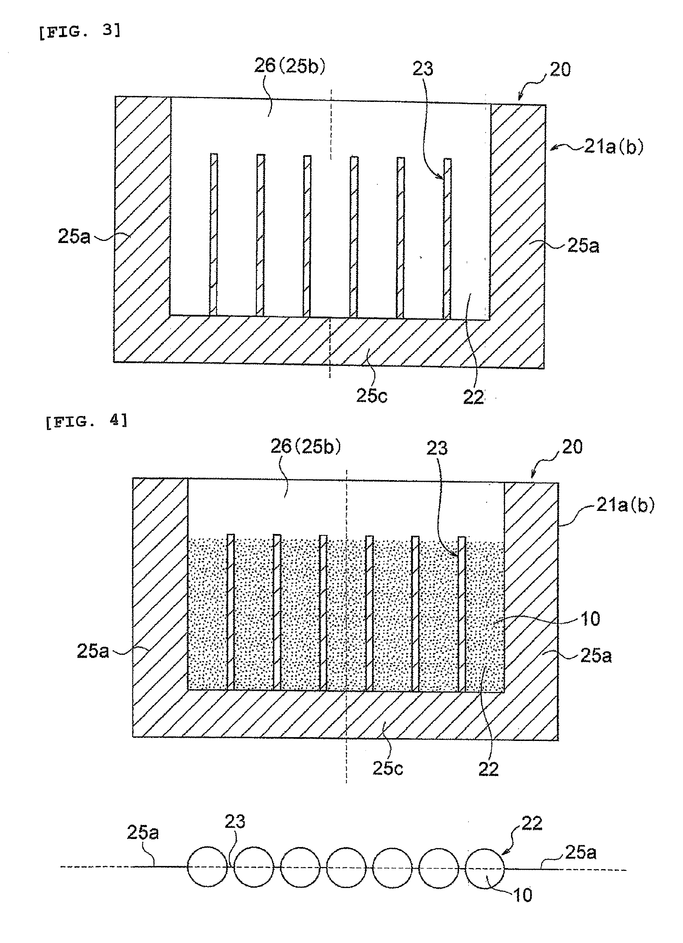

Next, description is made of a method of manufacturing the body temperature regulation pack according to this embodiment with reference to FIGS. 3 to 10.

As illustrated in FIG. 3, the two sheets 21a and 21b are overlapped with each other, and while leaving one side (upper edge portion 25b) which becomes an opening portion 26 for injecting the solid- liquid phase change material 10, the other three sides (bilateral edge portions 25a and lower edge portion 25c) are welded, to thereby form the inner pack 20. In the embodiment of this application, the two sheets 21a and 21b are bonded together. However, a single sheet may be folded at the center, and then the surrounding edge portions may be bonded together.

Next, the two sheets 21a and 21b are welded at necessary positions so that a region between the pair of opposing bilateral edge portions 25a are divided at regular intervals in a parallel direction. Thus, the plurality of elongated bag- like portions 22 are formed. Note that, the weldedportions 23 are provided at regular intervals in the embodiment of this application, but by changing the intervals as appropriate, the sizes of the respective elongated bag- like portions 22 may be adjusted.

Next, as illustrated in FIG. 4, by using the unwelded upper edge portion 25b as the opening portion 26, the solid- liquid phase change material 10 in the liquid phase is injected into each of the elongated bag- like portions 22 up to a height that does not exceed the upper end of the welded portion 23. At this time, the sectional shape of the elongated bag- like portion 22 when viewed from above becomes a circle which is more spindle- like than a true circle. The entire elongated bag- like portion 22 has a substantially columnar shape, and along the welded edge portions 25a, 25b, and 25c and the welded portions 23, wrinkles are formed on the surfaces of the elongated bag- like portions 22.

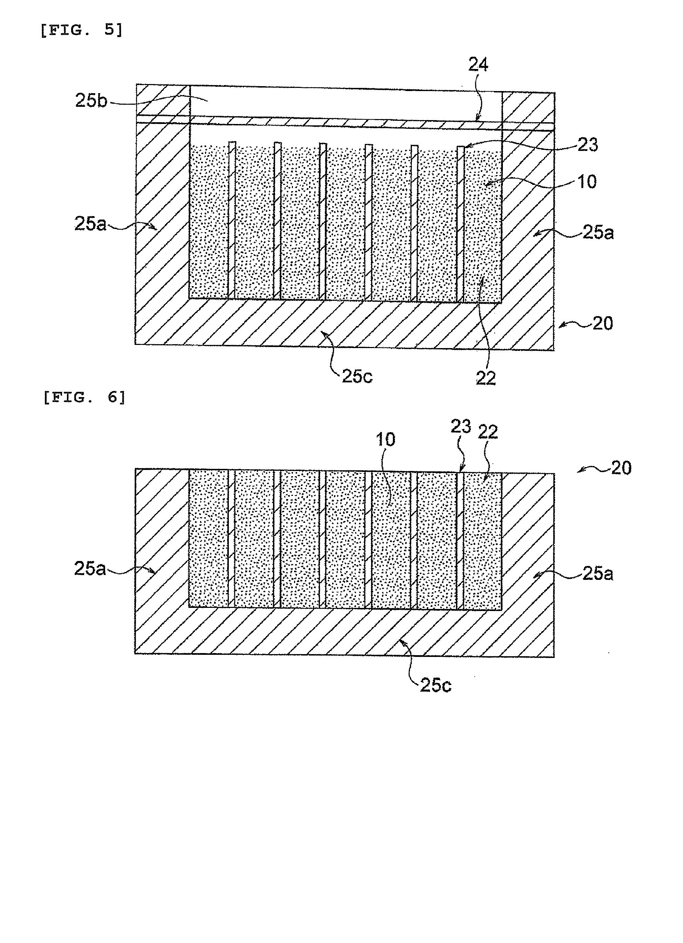

Next, the solid- liquidphase change material 10 filling the elongated bag- like portion 22 is cooled to become the solid phase. After that, as illustrated in FIG. 5, the interior of the inner pack 20 is sealed in vacuum, and the upper edge portion 25b which has been the opening portion 26 is welded for sealing. At this time, a welded portion 24 along the upper edge portion 25b is provided slightly above the welded portion 23 in a separated manner. After that, as illustrated in FIG. 6, the upper edge portion 25b is folded in order to fill each of the elongated bag- like portions 22 with the solid- liquid phase change material 10 without a gap.

Next, heat is applied to the solid- liquid phase change material 10 sealed in the inner pack 20 so that the phase returns to the liquid phase again. Then, as illustrated in FIGS. 6 and 7, all of the surrounding edge portions 25 are folded on one side (side on which the heat insulating material 30 (described later) is to be arranged), to thereby obtain a state in which the interior of the elongated bag- like portion 22 is filled with the solid- liquid phase change material 10 in the liquid phase without a gap. The side on which the edge portions are folded is the side opposite to the side on which the body temperature regulation pack 1 comes into contact with the body. This is because the action and effect of cooling are weakened if the folding comes to the body side. Note that, "filling without a gap" as referred to herein represents a state in which nearly 100% of the capacity of the elongated bag- like portion 22 is filled with the solid- liquid phase change material 10. Even when the body temperature regulation pack 1 is arranged in the vertical direction under a state in which the solid- liquid phase change material 10 is in the liquidphase, the elongatedbag- like portion 22 is not deformed.

Next, as illustrated in FIG. 8, on the one surface of the inner pack 20 (surface opposite to the surface at which the body temperature regulation pack 1 comes into contact with the body), the heat insulating material 30 is fixed so as to cover the entire part filled with the solid- liquid phase change material 10.

Next, as illustrated in FIG. 9, inside the outer bag- like portion 41 of the outer pack 40 formed of the two outer sheets 43a and 43b, the inner pack 20 having the heat insulating material 30 fixed thereon is inserted. Then, vacuum sealing is performed as illustrated in FIG. 10 so that the body temperature regulation pack 1 is completed. Note that, it is also possible to, after the inner pack 20 is sandwiched between the outer sheets 43a and 43b, bond together the circumferences of the outer sheets 43a and 43b to form the outer pack 40, and then perform the vacuum sealing. Further, in this embodiment, the two sheets 43a and 43b having the same shape and made of the same material are used, but a single sheet may be folded to form a pair, and the inner pack 20 may be sandwiched therebetween.

The body temperature regulation pack 1 formed as described above includes the plurality of elongated bag-like portions 22 arranged in the parallel direction and arrayed in parallel to each other through intermediation of the welded portions 23 having flexibility. Therefore, even when the solid-liquid phase change material 10 is in the solid phase, as illustrated in FIG. 11, the body temperature regulation pack 1 can be folded along the shape of the body due to the welded portions 23 serving as joints. In this manner, the degree of contact of the body temperature regulation pack 1 with the human body is further increased. Therefore, the body temperature regulation pack 1 satisfies both of high heat absorption ability and high heat releasing ability, which is obtained by providing the 100% solid-liquid phase change material 10 as the cooling material or the heat retaining material, and the following property to the human body. Further, the column-shaped elongated bag-like portions are arrayed in parallel to each other in the lateral direction, and hence a substantially plate shape having a surface with a concavo-convex shape is obtained as a whole. Therefore, when the plurality of body temperature regulation packs 1 are stored, as illustrated in FIG. 12, the body temperature regulation packs 1 can be stably stored in an overlapping manner, and no extra space is required.

(Second Embodiment)

Next, with reference to FIGS. 13 to 21, description is made of a body temperature regulation pack 100 according to another embodiment of the present invention, which is used by being attached to the pack holder 2 to be described later. The body temperature regulation pack 100 has an outer shape in the same form as the body temperature regulation pack 1 illustrated in FIG. 1. The entirety is formed into a substantially tabular rectangular shape, and the body temperature regulation pack 100 includes a plurality of bag members T arranged in parallel to each other.

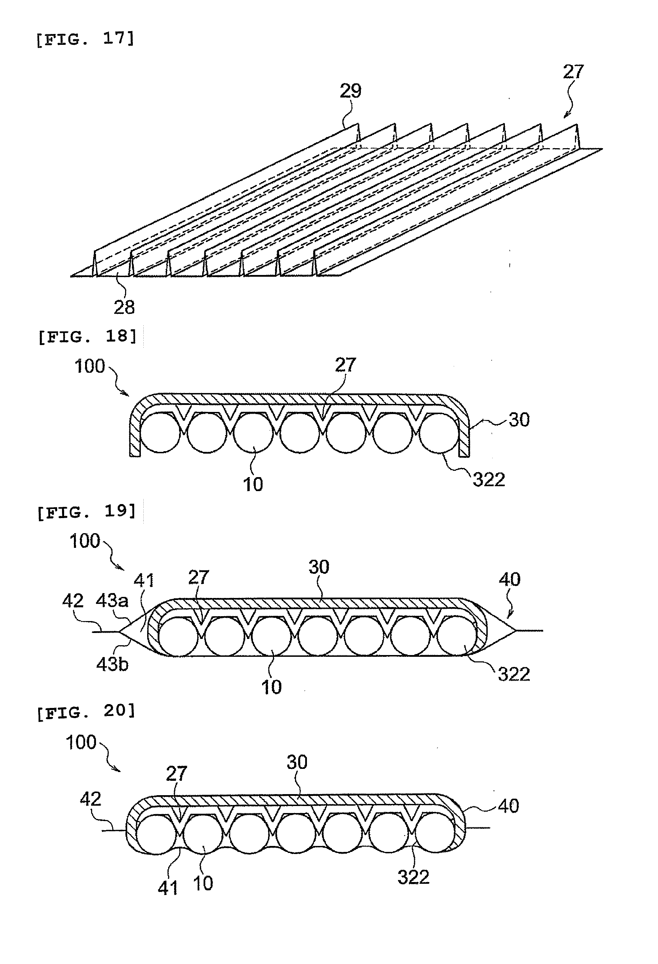

As illustrated in FIG. 20, the body temperature regulation pack 100 includes a plurality of elongated bag members 322 each filled with the solid- liquid phase change material 10, an elongated bag member arraying member 27 for arraying the elongated bag members 322 in parallel to each other, the heat insulating material 30 for covering one surface of the plurality of elongated bag members 322 arrayed in parallel to each other in the parallel direction, and the outer pack 40 for covering the elongated bag members 322, the elongated bag member arraying member 27, and the heat insulating material 30 as a whole. Further, the surface of the body temperature regulation pack 100 has a concavo- convex shape along the shape of the elongated bag members inside the outer pack 40. Note that, in this embodiment, the body temperature regulation pack 100 is formed into a substantially tabular rectangular shape as a whole, but the present invention is not limited thereto, and the body temperature regulation pack 100 may have another shape.

As illustrated in FIGS. 13 to 15, each of the elongated bag members 322 is formed of a film to have an elongated cylindrical shape whose both ends are welded. Similarly to the inner sheet 21 of the first embodiment, the film is preferred to have a three-layer structure in which a sheet-like polyethylene, aluminum foil, and a sheet-like nylon (trademark) are bonded together in order to secure flexibility and strength. Note that, as long as the film of the elongated bag member 322 is flexible and has sufficient strength and the solid-liquid phase change material 10 sealed therein does not leak out, the materials are not particularly limited to those in this embodiment. Further, the length and the thickness of the elongated bag member 322 may be changed as appropriate in accordance with the application and the attaching region.

With use of the plurality of independent elongated bag members 322 as in this embodiment, as compared to the case where the pair of inner sheets 21a and 21b are used to form the plurality of elongated bag-like portions 22 as in the first embodiment, stress to the film of each of the elongated bag members 322 is reduced, and durability is increased. That is, when the body temperature regulation pack 100 is curved as illustrated in FIG. 11, the inner sheet 21 receives a pulling force during the curving in the first embodiment. However, if the elongated bag members 322 are independent from each other as in this embodiment, even when the body temperature regulation pack 100 is curved along the body region, an excess stress is not applied. Therefore, the risk that the film is damaged and the function of the body temperature regulation pack 100 is lost is minimized. That is, the risk that the aluminum layer for securing the water vapor barrier property of the film is damaged is minimized. Further, even if by chance a needle-like object pierces the film from the outside, only one of the elongated bag members 322 damaged with an opened pinhole loses its function. Therefore, the other elongated bag members 322 are not affected, and the function of the body temperature regulation pack 100 as a whole is not lost. That is, the body temperature regulation pack 100 includes the plurality of independent elongated bag members 322, and hence the risk of loss of function is minimized. Further, merely by replacing the damaged elongated bag member 322, the function of the body temperature regulation pack 100 may be regenerated, and hence repair is easy and inexpensive. Still further, by manufacturing a plurality of types of the elongated bag members 322 having different lengths and thicknesses in advance, the elongated bag members 322 may be combined as appropriate when the body temperature regulation pack 100 is manufactured. In this manner, more appropriate body temperature regulation becomes possible.

Inside the elongated bag member 322, the solid-liquid phase change material 10 is filled in the elongated bag member 322 to capacity without a gap, to thereby form the elongated bag member 322 into an elongated columnar shape. Therefore, even when the body temperature regulation pack 100 is arranged in a vertical direction in a state in which the solid-liquid phase change material 10 is in a liquid phase, there is no margin for the elongated bag member 322 to generate a deflection, and hence the elongated bag member 322 is not deformed. Further,the pluralityofindependent elongated bag members 322 are arranged in the parallel direction, and hence even when the solid-liquid phase change material 10 changes into the solid phase, the body temperature regulation pack 100 can be folded along the shape of the body. Therefore, a degree of contact of the body temperature regulation pack 100 with the human body is increased. Further, the column-shaped elongated bag members 322 are arrayed in parallel to each other in the lateral direction, and hence a substantially tabular shape having a surface with a concavo-convex shape is obtained as a whole. Therefore, when the plurality of body temperature regulation packs 100 are stored, the body temperature regulation packs 100 can be stably stored in an overlapping manner, and no excess space is required. Note that, "filling without a gap" as referred to herein represents a state in which, similarly to the first embodiment, nearly 100% of the capacity of the elongated bag member 322 is filled with the solid-liquid phase change material 10. Even when the body temperature regulation pack 100 is arranged in the vertical direction under a state in which the solid-liquid phase change material 10 is in the liquid phase, the elongated bag member 322 is not deformed.

Further, similarly to the first embodiment, in the temperature regulation pack 100, there is no need to worry about deformation even when the amount of the solid- liquid phase change material 10 is increased. Therefore, as illustrated in FIG. 22, by changing the diameter of the elongated bag member 322 as appropriate, the amount of the solid- liquidphase change material 10 can be adjusted in accordance with the body region and the necessary cooling function retention time or temperature retaining function retention time.

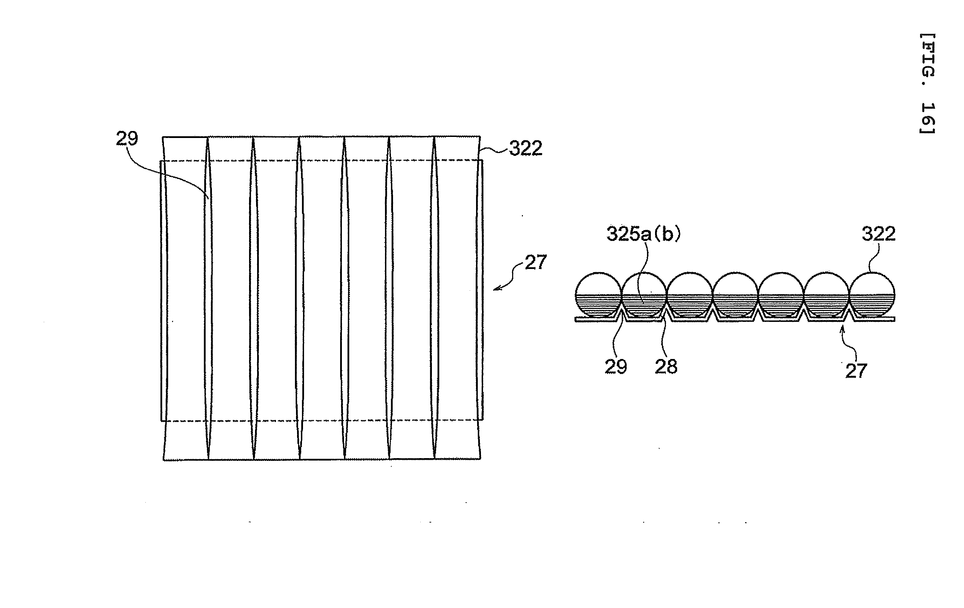

As illustrated in FIGS. 16 and 17, the elongated bag member arraying member 27 for placing and arraying the elongated bag members 322 has a rectangular shape which covers substantially the entire length of the elongated bag members 322 when viewed from above. Further, when viewed from the lateral side, the elongated bag member arraying member 27 includes pocket portions 28 in which the respective elongated bag members 322 are placed, and projecting portions 29 which project between the adjacent elongated bag members 322, for arranging the respective elongated bag members 322 at positions at regular intervals. Further, the elongated bag member arraying member 27 is made of a flexible material such as cardboard, and can be freely curved along the body region. Further, the shape of the elongated bag member arraying member 27 is not limited to that in this embodiment, and one or a plurality of narrow piece members may be transversely provided for use.

As illustrated in FIG. 16, by placing the elongated bag members 322 on the elongated bag member arraying member 27, the positions of the elongated bag members 322 can be held at regular intervals. As a result, the risk that the elongated bag members 322 hit each other to be scratched is eliminated. Further, the elongated bag member arraying member 27 is made of a material having flexibility, and hence the elongated bag member arraying member 27 can be curved so as to maintain a degree of the contact of the body temperature regulation pack 100 with the body region.

The characteristics of the solid-liquid phase change material 10, the heat insulating material 30, and the outer pack 40 are the same as those in the first embodiment, and hence description thereof is omitted.

Next, a method of manufacturing the body temperature regulation pack 100 of this embodiment is described with reference to FIGS. 13 to 21.

First, as illustrated in FIG. 13, a film is used to form the cylindrical elongated bag member 322. One end portion thereof is opened as an injection port 326 for the solid- liquid phase change material, and an end portion on the opposite side is welded. The diameter and the length of the elongated bag member 322 may be changed as appropriate in accordance with the application.

Next, as illustrated in FIG. 14, the solid- liquid phase change material 10 in the liquid phase is injected from the injection port 326 under vacuum to fill the elongated bag member 322. After that, the injection port 326 is sealed by welding to obtain a state in which the elongated bag member 322 is filled with the solid- liquid phase change material 10 without a gap. When vacuum sealing is performed as described above, it is unnecessary to fill the elongated bag member 322 with the solid- liquid phase change material 10 in the liquidphase, perform vacuum sealing with the cooled solid- liquid phase change material 10 in the solid phase state, and then return the phase to a liquid phase again. Accordingly, the manufacturing process is shortened, and mass production becomes possible. Note that, this vacuum sealing operation can be performed with use of a known automatic vacuum packing machine, but the description of the packing machine is omitted herein.

Next, as illustrated in FIG. 15, welded portions 325a and 325b at both ends are folded toward one side of the elongated bag member (side on which the elongated bag member arraying member 27 and heat insulating material 30 (described later) are to be arranged) . The side on which the welded portions 325a and 325b are folded is the side opposite to the side on which the body temperature regulation pack 100 comes into contact with the body. This is because the action and effect of cooling are weakened if the folded part comes to the body side.

Further, the plurality of elongated bag members 322 formed by the above- mentioned manufacturing method are placed on the elongated bag member arraying member 27 so as to be arrayed in parallel to each other in the parallel direction at regular intervals (see FIG. 16) . The side on which the elongated bag member arraying member is mounted is the side on which the welded portions 325a and 325b are folded, that is, the side opposite to the side on which the body temperature regulation pack 100 comes into contact with the body. This is for preventing the heat from the outside from transferring to the body temperature regulation pack 100.

Next, as illustrated in FIG. 18, on a surface on a side opposite to a surface at which the body temperature regulation pack 100 comes into contact with the body, the heat insulating material 30 is fixed so as to cover the entire plurality of elongated bag members 322 together with the elongated bag member arraying member 27. At this time, the side on which the elongated bag member arraying member 27 is fixed is covered with the heat insulating material 30. The reason is as follows. If the elongated bag member arraying member 27 is present on the side on which the heat insulating material 30 is absent, that is, the side which comes into contact with the body, the cooling efficiency or the heat retaining efficiency is degraded.

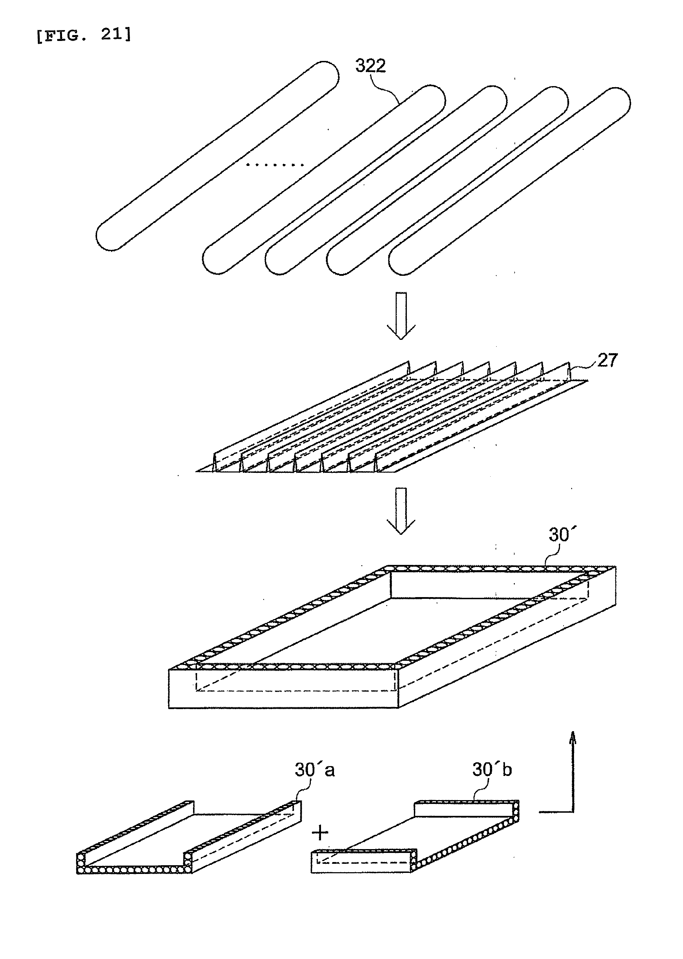

Alternatively, as the heat insulating material, there may be used a box- shaped heat insulating material 30' which has an opened upper surface as illustrated in FIG. 21. When this heat insulating material 30' is used, the elongated bag member arraying member 27 is placed inside the heat insulating material 30', and the elongated bag members 322 are placed and arrayed thereon. With this, the elongated bag member arraying member 27 and the heat insulating material 30' can be more easily fixed to the elongated bagmembers322. Further, as a result of fixing of the heat insulating material 30' as described above, regarding the plurality of elongated bag members 322 as a whole, one side which does not come into contact with the body and the four side portions can be covered with the heat insulating material 30', and thus the heat insulating effect is improved. Note that, the heat insulating material 30' is formed by combining, into a box shape, two rectangular heat insulating panels 30'a and 30'b each having erected portions at both ends. As a result, the heat insulating material is doubled at the bottom surface, and thus a higher heat insulatingperformance can be obtained. Note that, the method of forming the box- shaped heat insulating material 30' is not limited thereto, and a single heat insulating panel may be processed for formation. Further, the heat insulating panels 30'a and 30'b are each formed of a tabular heat insulating material obtained by bonding an aluminum foil sheet on each of both surfaces of a known bubble wrap, but the material is not limited thereto.

Next, as illustrated in FIG. 19, inside the outer bag- like portion 41 of the outer pack 40 formed of the two outer sheets 43a and 43b, the elongated bag members 322 having the elongated bag member arraying member 27 and the heat insulating material 30 (30') fixed thereon are inserted. Then, vacuum sealing is performed as illustrated in FIG. 20 to complete the body temperature regulation pack 100. Note that, it is also possible to, after the elongated bag members 322 are sandwiched between the outer sheets 43a and 43b, bond together the circumferences of the outer sheets 43a and 43b to form the outer pack 40, and then perform the vacuum sealing. Further, in this embodiment, the two sheets 43a and 43b having the same shape and made of the same material are used, but a single sheet may be folded to form a pair, and the elongated bag members 322 may be sandwiched therebetween.

The body temperature regulation pack 100 formed as described above includes the plurality of independent elongated bag members 322 arrayed in parallel to each other in the parallel direction. Therefore, even when the solid-liquid phase change material 10 is in the solid phase, the body temperature regulation pack 100 can be folded along the shape of the body. In this manner, the degree of contact of the body temperature regulation pack 100 with the human body is further increased. Therefore, the body temperature regulation pack 100 satisfies both high heat absorption ability or high heat releasing ability, which is obtained by providing 100% of the solid-liquid phase change material as the cooling material or the heat retaining material, and the following property to the human body. Further, the column-shaped elongated bag members 322 are arrayed in parallel to each other in the lateral direction, and hence a substantially tabular shape having a surface with a concavo-convex shape is obtained as a whole. Therefore, when the plurality of body temperature regulation packs 100 are stored, as illustrated in FIG. 12, the body temperature regulation packs 100 can be stably stored in an overlapping manner, and no extra space is required.

Note that, in this embodiment, the number of elongated bag members 322 forming the body temperature regulation pack 100 is seven. However, as long as the body region which requires body temperature regulation can be covered, the number is not limited thereto. Further, the thickness and the length of the elongated bag member 322 may be freely set in accordance with the application.

[Mesh Bag]

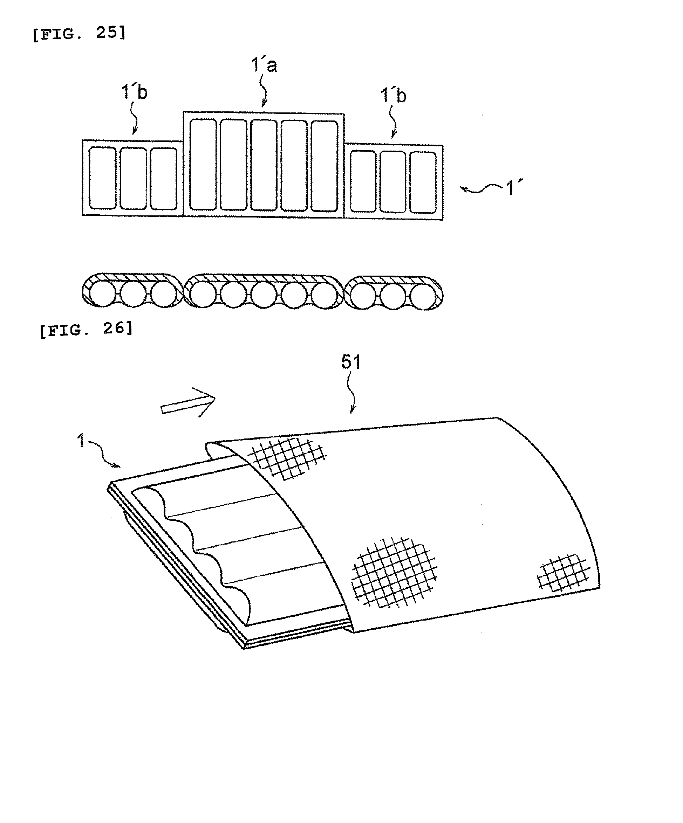

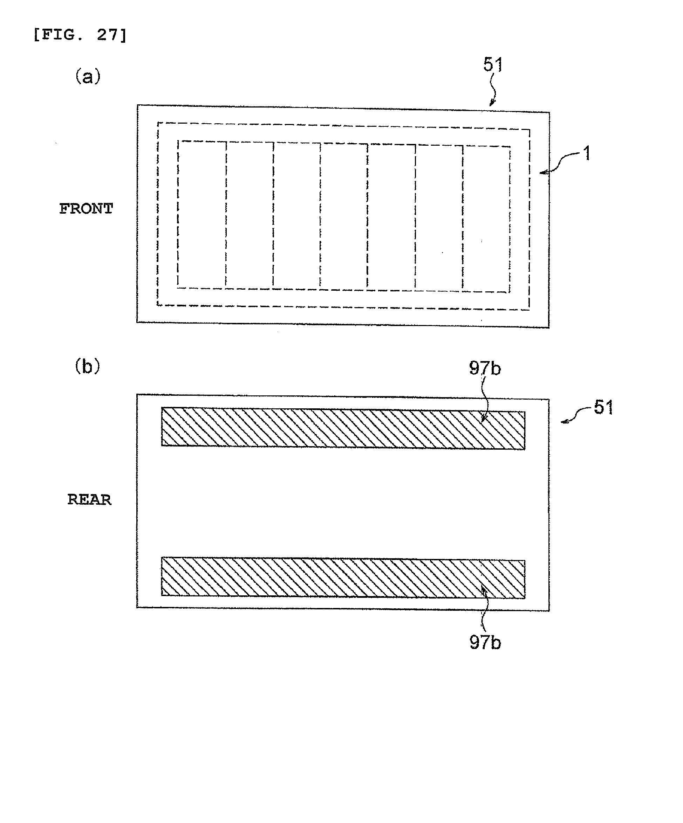

Next, with reference to FIGS. 26 and 27, description is made of a mesh bag 51 made of a mesh material, into which the above- mentioned body temperature regulation pack 1 or 100 is inserted and which is dedicated for holding the above- mentioned body temperature regulation pack 1 or 100 to a torso pack holder 80 (see FIG. 28) to be described later. The body temperature regulation packs 1 and 100 have the same outer form, and hence, for the sake of convenience, description is made with reference to the body temperature regulation pack 1. Note that, the outer pack 40 of the body temperature regulation pack 1 is made of a film as described above, and hence a surface fastener cannot be directly fixed to nylon (trademark) on the surface. A surface fastener 97b can be directly fixed to nylon (trademark) only with the use of an adhesive. Welding with a solvent is most efficient, but the film strength of the outer pack 40 is reduced accordingly. Therefore, in view of the above- mentioned point, as illustrated in FIG. 26, the body temperature regulation pack 1 is inserted into the mesh bag 51 so that the body temperature regulation pack 1 can be removably attached to the torso pack holder 80.

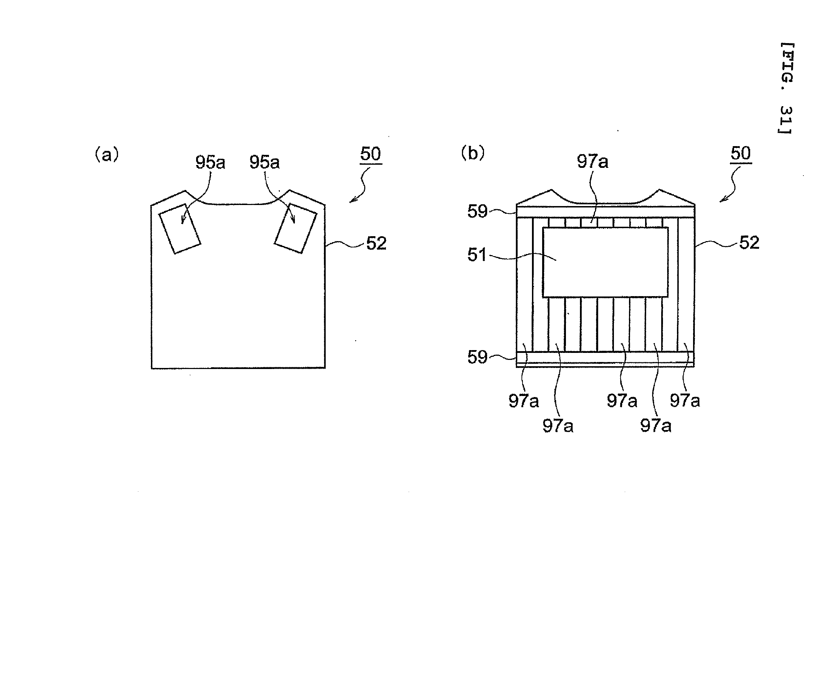

As illustrated in FIGS. 26 and 27, the mesh bag 51 to be arranged at the chest of the wearer has a rectangular shape, and is provided with the surface fasteners 97b on a surface on a side to be attached to the torso pack holder 80 so that the mesh bag 51 can be removably attached to surface fasteners 97a (see FIGS. 31) of the torso pack holder 80 to be described later. In this embodiment, the mesh bag 51 has a rectangular shape, but shapes other than the rectangular shape, such as an elliptical shape, may be adopted in accordance with the body region to which the mesh bag 51 is attached or the shape of the body temperature regulation pack 1 to be inserted.

In order to removably attach the body temperature regulation pack 1 to the torso pack holder 80, as illustrated in FIG. 26, the body temperature regulation pack 1 is inserted into the mesh bag 51, and an insertion port is openably closed by known means such as a surface fastener. The body temperature regulation pack 1 can be freely taken out from the mesh bag 51, and hence in accordance with the application or the like, the type of the body temperature regulation pack 1 (for example, one that contains a different amount of the sealed solid-liquid phase change material) can be replaced. Further, even when the mesh bag 51 is stained, only the body temperature regulation pack 1 need be taken out to be washed. Note that, in this embodiment, as illustrated in FIG. 27 (a), the mesh bag 51 contains a single body temperature regulation pack 1, but depending on the capacity of the mesh bag 51 and the size of the body temperature regulation pack 1, the mesh bag 51 may contain two or more body temperature regulation packs 1. Further, in this embodiment, the insertion port can be freely opened and closed, but the insertion port may be completely closed by means such as sewing.

As illustrated in FIG. 27(b), the surface fasteners 97b are each formed into an elongated piece, and are provided in parallel to each other at a pair of endportions of the mesh bag 51, respectively. Note that, the number of surface fasteners is not limited to that in this embodiment. As long as the body temperature regulation pack 1 can be held in the torso pack holder 80, the number of surface fasteners may be one, or three or more. Further, one surface of the mesh bag may be entirely formed into a surface fastener, or the material of the mesh bag itself may be a surface fastener. Further, the surface fastener 97a on the torso pack holder 80 side to be described later is a loop-shaped surface fastener so as to prevent fastening to other clothing. The surface fastener of the mesh bag 51 is a hook-shaped surface fastener. Note that, the present invention is not limited thereto, and the surface fastener 97b of the mesh bag 51 may be a loop-shaped surface fastener, and the surface fastener 97a on the torso pack holder 80 side may be a hook-shaped surface fastener.

The material of the mesh bag 51 is mesh fabric. With this, heat released from the body can be absorbed more efficiently. Further, when the body temperature regulation pack 1 for cooling is immersed in ice water so that the phase returns to the solid phase, in the case of the mesh bag 51 made of a mesh material, it is possible to cool the entire mesh bag 51 by immersing it in ice water without having to take out the body temperature regulation pack 1 all the time.

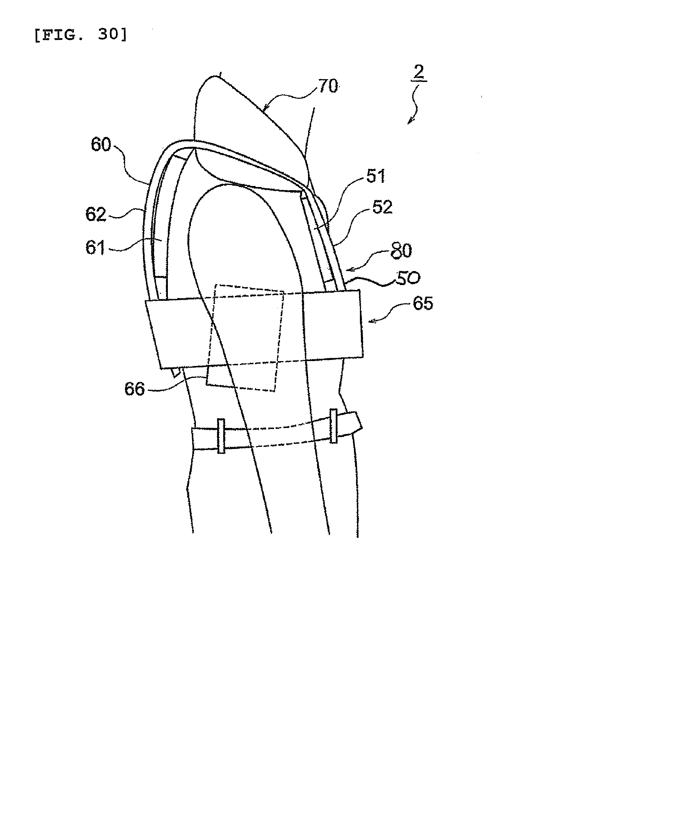

In the above, description has been made of the mesh bag 51 to be arranged to the chest, but a mesh bag 61 to be arranged to the back and a mesh bag 66 to be arranged under the arm are similarly configured (see FIG. 30).

[Pack Holder]

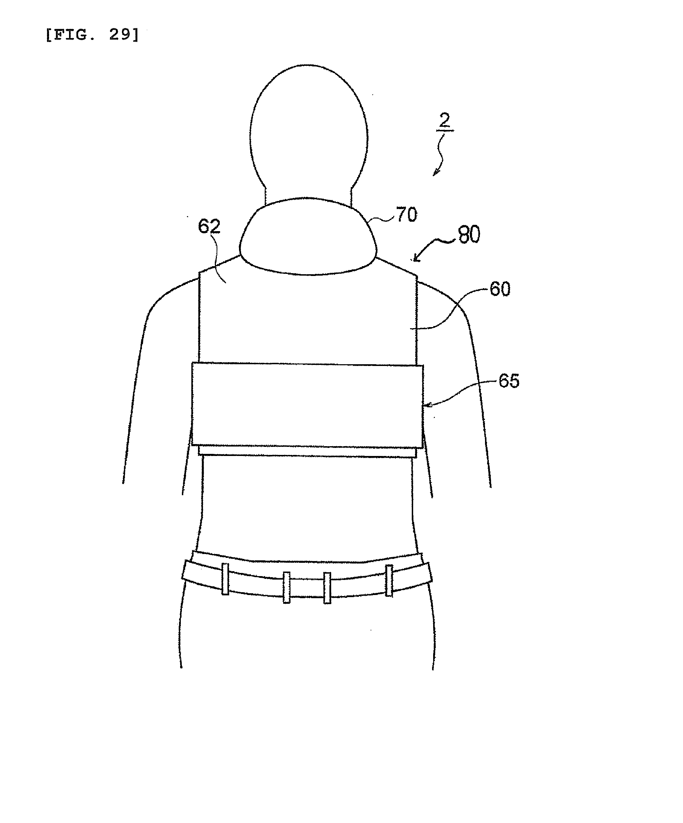

With reference to FIGS. 28 to 35, description is made of the pack holder 2 for wearing the above-mentioned body temperature regulation pack 1 on the body. The pack holder 2 includes the torso pack holder 80 for wearing the body temperature regulation pack 1 on the torso, and a collar pack holder 70 for wearing g the body temperature regulation pack 1 around the neck. The pack holder 2 further includes a leg pack holder 90 for wearing the body temperature regulation pack 1 on the legs.

<Torso Pack Holder>

First, with reference to FIGS. 28 to 33, description is made of the torso pack holder 80 dedicated for wearing the body temperature regulation pack 1 mainly on the torso.

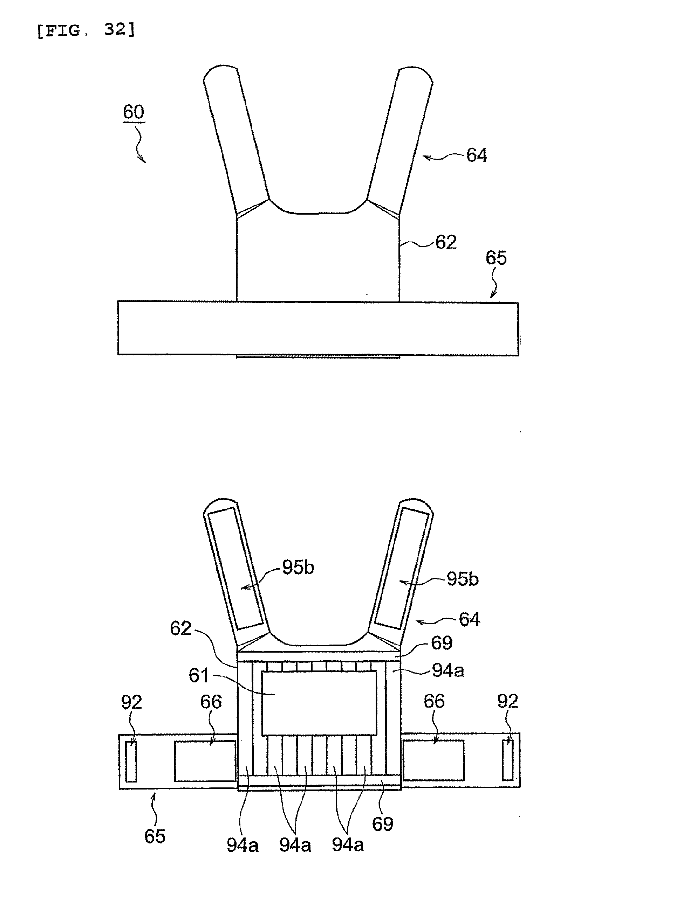

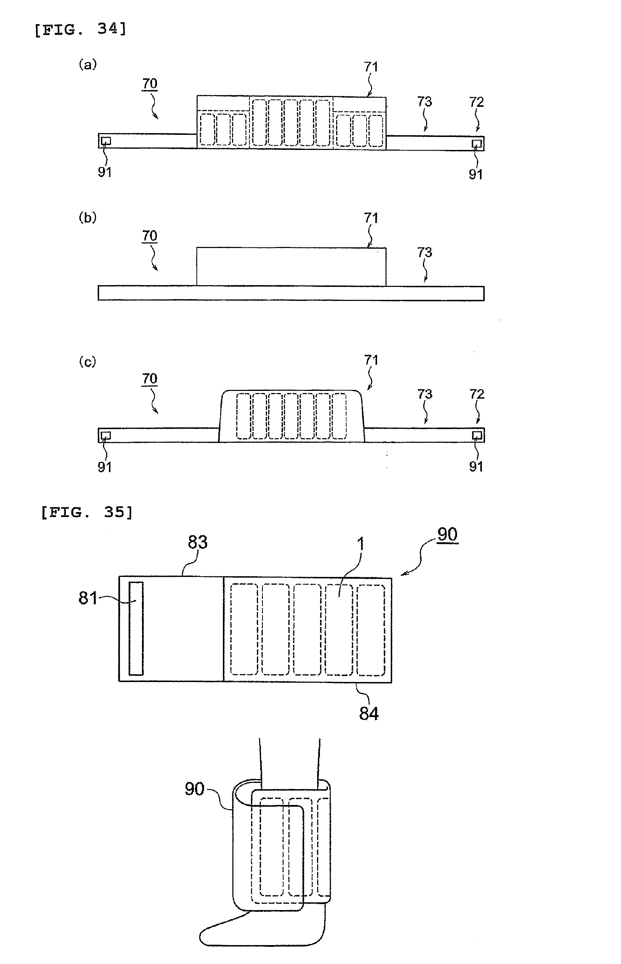

FIGS. 28 to 30 are a front view, a rear view, and a side view, respectively, illustrating a wearing state of the torso pack holder 80 of the present invention. Note that, in FIGS. 28 to 30, in addition to the torso pack holder 80 worn on the torso, the wearing state of the collar pack holder 70 (see FIGS. 34) (to be described later) worn around the neck is illustrated. FIGS. 31 and 32 illustrate respective parts of the torso pack holder 80 of the present invention in an exploded manner, and are views illustrating the front side and the rear side of a foreside portion and the front side and the rear side a backside portion, respectively. Note that, the front surface as referred to herein corresponds to a surface on a side opposite to a side which comes into contact with the body when the torso pack holder 80 is worn, and the rear surface corresponds to a surface on the side which comes into contact with the body. The same is true in the following description as well.

First, description is made of the overall structure of the torso pack holder 80. As illustrated in FIGS. 28 to 30, the torso pack holder 80 includes a foreside portion 50 for covering the chest, and a backside portion 60 for covering a region from the shoulder to the back. On the lower portion of the front surface of the backside portion 60, a band portion 65 for covering a region under the arm is integrally sewn. The backside portion 60 includes shoulder portions 64 at both shoulder parts, and the foreside portion 50 is removably connected to the backside portion 60 through intermediation of the shoulder portions 64. The foreside portion 50 and the backside portion 60 are separated from each other so as to be removably worn. Therefore, the torso pack holder 80 can be adjusted depending on the build of the wearer and the number of the body temperature regulation packs 1 to be arranged. Note that, in this embodiment, the torso pack holder 80 covers only the chest and the upper half of the back, but the present invention is not limited thereto. The length of the torso pack holder 80 may be changed to cover the abdomen and the lower half of the back.

In both the foreside portion 50 and the backside portion 60, the main material of the torso pack holder 80 is mesh fabric. By employing mesh fabric as the material, the entire weight of the torso pack holder 80 can be reduced, and heat released from the body of the wearer is prevented from staying inside the torso pack holder 80. Further, when the solid-liquid phase change material 10 for cooling is used, because the torso pack holder 80 is made of a mesh material which can be quickly dried even when getting wet, the entire torso pack holder 80 can be cooled by being immersed in ice water, which enables the solid-liquid phase change material 10 in the liquid phase to easily return to a solid phase in a short period of time. Further, through the mesh fabric, water and air can easily flow around the entire torso pack holder 80. Therefore, when the entire torso pack holder 80 is cooled by being immersed in water or cooled in the refrigerator, the function of the solid-liquid phase change material 10 can be recovered faster.

As illustrated in FIGS. 31, the foreside portion 50 includes a body temperature regulation pack holding portion 52 for covering the chest of the wearer, and the mesh bag 51 is attached to the rear surface of the body temperature regulation pack holding portion 52. As illustrated in FIG. 31 (a), on the front surface of the body temperature regulation pack holding portion 52, surface fasteners 95a which engage with surface fasteners 95b of the backside portion 60 to be described later are provided at both shoulder parts, respectively. With this, the foreside portion 50 is removably attached to the backside portion 60, to thereby form the torso pack holder 80. Further, the surface fasteners 95a can be fastened to the surface fasteners 95b while shifting the engagement positions as appropriate. In this manner, the position of the foreside portion 50 with respect to the body and the degree of contact with respect to the body can be adjusted. Further, with the band portion 65 described later, the foreside portion 50 is fixed at the lower part of the chest or the abdomen (see FIGS. 28 to 30). Note that, the engagement means for the foreside portion 50 and the backside portion 60 is not limited to the surface fasteners, and any means may be employed as long as the means can be freely disengaged and can appropriately adjust the degree of contact of the torso pack holder 80 with the body, such as engagement obtained by fixing belts whose lengths are adjustable to both the shoulder parts, and combining a tongue fixed to the end of each of the belts and a buckle.

As illustrated in FIG. 31(b), on the rear surface of the body temperature regulation pack holding portion 52, a plurality of elongated surface fasteners 97a, in this embodiment, six surface fasteners 97a, are uniformly arranged from the left end to the right end in parallel to each other at regular intervals in the vertical direction. Further, on the upper and lower sides of the rear surface of the body temperature regulation pack holding portion 52, a pair of reinforcing tapes 59 as elongated pieces are provided in the lateral direction. The foreside portion 50 is mainly made of mesh fabric, and hence is not firm. Therefore, the reinforcing tapes 59 are used to reinforce the foreside portion 50. Further, the reinforcing tape 59 also has a function of processing the terminals of the surface fasteners 97a sewn in the vertical direction. In the mesh bag 51, the surface fasteners 97b (see FIG. 27(b)) on the rear surface are fixed in a direction intersecting with the surface fasteners 97a of the body temperature regulation pack holding portion 52. Therefore, along the surface fasteners 97a of the body temperature regulation pack holding portion 52, the fastening positions of the surface fasteners 97b of the mesh bag 51 can be changed as appropriate on upper, lower, left, and right sides (mainly, in the up-down direction). Thus, the mesh bag 51 can be removably attached to a necessary position. Further, the surface fasteners 97a are uniformly provided from the left end to the right end. Therefore, instead of providing only one mesh bag 51 as in this embodiment, two mesh bags 51 may be provided on the left and right sides, or a plurality of mesh bags 51 may be arranged on the left, right, upper, and lower sides. In this manner, the degree of freedom in arrangement of the body temperature regulation packs 1 is increased. Further, for example, on a fire fighting site, the oxygen level may not be secure, and hence it is necessary for the worker to carry an air respirator on his/her back as a backpack. In this case, the chest belt must be fastened, and hence only one body temperature regulation pack 1 can be attached to the center part of the chest. On the other hand, in the case of work in a controlled area of a nuclear power plant in which the oxygen level is secure, it is unnecessary to wear an air respirator, and only a gas/dust mask is generally worn. In this case, while it is unnecessary to carry an air respirator on his/her back with the chest belt, continuous work for a long period of time, which may reach three hours, is required. Therefore, in such cases, there is no restriction in space for arranging the body temperature regulation packs 1. By wearing two or more body temperature regulation packs 1 having high heat absorption performance on the left and right sides, the work becomes safer. Further, the surface fastener exhibits the engagement action when hook-shapedsurfacefastenersandloop-shapedsurfacefasteners are provided in pairs. The surface fastener 97a of the body temperature regulation pack holding portion 52 is preferably a loop-shaped surface fastener. The reasons are as follows. When a hook-shaped surface fastener is provided on a surface which comes into contact with the body, the hook-shaped surface fastener may unnecessarily fasten to clothing or the like worn under the torso pack holder 80. In addition, the hook-shaped surface fastener feels rough. However, the present invention is not limited thereto, and the surface fastener 97a of the body temperature regulation pack holding portion 52 may be a hook-shaped surface fastener, and the surface fastener 97b of the mesh bag 51 may be a loop-shaped surface fastener. Note that, the surface fasteners 97a may be arrayed in the lateral direction. In this case, the surface fasteners 97b of the mesh bag 51 may be arranged in vertical direction. Further, six surface fasteners 97a are used in this embodiment, but the number of surface fasteners 97a is arbitrary as long as the mesh bag 51 can appropriately be held and mounted. Still further, the surface fastener 97a is not limited to a plurality of fasteners having an elongated shape, and the entire rear surface of the body temperature regulation pack holding portion 52 may be formed into a surface fastener.

As illustrated in FIG. 32, the backside portion 60 includes a body temperature regulation pack holding portion 62 to which a mesh bag 61 is attached, and the pair of shoulder portions 64 for supporting the torso pack holder 80 at both shoulders. As illustrated in FIG. 32(b), on the rear surface of the body temperature regulation pack holding portion 62, a plurality of elongated surface fasteners 94a, in this embodiment, six surface fasteners 94a, are uniformly arranged from the left end to the right end in parallel to each other at regular intervals in the vertical direction. Further, on the upper and lower sides of the rear surface of the body temperature regulation pack holding portion 62, a pair of reinforcing tapes 69 as elongated pieces are provided in the lateral direction. The backside portion 60 is mainly made of mesh fabric, and hence is not firm. Therefore, the reinforcing tapes 69 are used to reinforce the backside portion 60. Further, the reinforcing tape 69 also has a function of terminating the surface fasteners 94a sewn in the vertical direction. In the mesh bag 61, the surface fasteners 94b (see FIG. 27(b)) on the rear surface are fixed in a direction intersecting with the surface fasteners 94a of the body temperature regulation pack holding portion 62. Therefore, along the surface fasteners 94a of the body temperature regulation pack holding portion 62, the fastening positions of the surface fasteners 94b of the mesh bag 61 can be changed as appropriate on upper, lower, left, and right sides (mainly, in the up-down direction). Thus, the mesh bag 61 can be removably attached to a required position. Further, the surface fasteners 94a are uniformly provided from the left end to the right end. Therefore, instead of providing only one mesh bag 61, two mesh bags 61 may be provided on the left and right sides, or a plurality of mesh bags 61 may be arranged on the left, right, upper, and lower sides. In this manner, the degree of freedom in arrangement of the body temperature regulation packs 1 is increased. Further, as for the surface fastener, similarly to the foreside portion 50, the surface fastener 94a of the body temperature regulation pack holding portion 62 is preferably a loop-shaped surface fastener. However, the present invention is not limited thereto, and the surface fastener 94a of the body temperature regulation pack holding portion 62 may be a hook-shaped surface fastener, and the surface fastener 94b of the mesh bag 61 may be a loop-shaped surface fastener. Note that, the surface fasteners 94a may be arrayed in the lateral direction. In this case, the surface fasteners 94b of the mesh bag 61 may be arranged in the vertical direction. Further, six surface fasteners 94a are used in this embodiment, but the number of surface fasteners 94a is arbitrary as long as the mesh bag 61 can be appropriately held and mounted. Still further, the surface fastener 94a is not limited to a plurality of fasteners having an elongated shape, and the entire rear surface of the body temperature regulation pack holding portion 62 may be formed into a surface fastener.

As illustrated in FIG. 32, the pair of shoulder portions 64 are provided so as to extend upward at parts of the body temperature regulation pack holding portion 62, which correspond to both the shoulders. On the rear surface of the shoulder portion 64, the square surface fastener 95b which engages with the surface fastener 95a of the foreside portion 50 is provided. With this, the foreside portion 50 and the backside portion 60 are coupled to each other in a manner that positions thereof are adjustable. Further, the surface fastener 95b is provided on the entire rear surface of the shoulder portion in the length direction. Therefore, by shifting the fastening positions of the surface fasteners 95a and 95b as appropriate, in accordance with the build of the wearer, the number of the body temperature regulation packs 1 to be placed, the thickness of the body temperature regulation pack 1, etc., the degree of contact of the torso pack holder 80 with the body can be adjusted, or the position to be covered with the torso pack holder 80 can be adjusted.

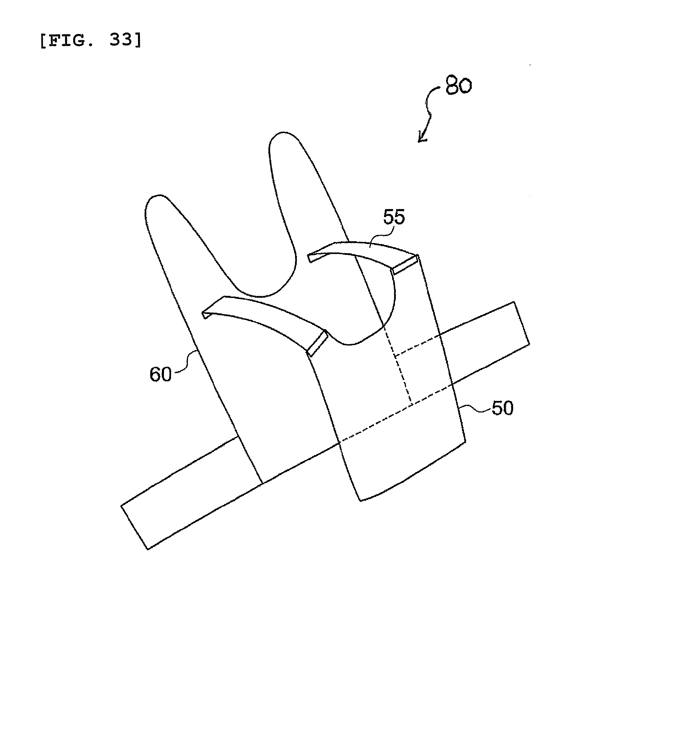

Alternatively, in order to facilitate the wearing, as illustrated in FIG. 33, the foreside portion 50 and the backside portion 60, which are independent from each other, may be connected to each other by two flat stretchable band- like members 55 (elastic bands, etc.) at parts of the foreside portion 50, which correspond to both the shoulders, and parts of the backside portion 60, which correspond to both the shoulders. Both ends of each of the band- like members 55 are sewn to the part of the foreside portion 50, which corresponds to both shoulders, and the part of the backside portion 60, which corresponds to both shoulders. Alternatively, holes may be provided in bilateral shoulder parts of each of the foreside portion 50 and the backside portion 60, and the band- like members 55 may be allowed to pass through the holes for connection. In this case, the wearer wears the torso pack holder 80 by inserting his/her head between the two band- like members 55. Under this state, with the shoulder portions 64, the distance between the foreside portion 50 and the backside portion 60 can be adjusted in accordance with build or the like.

As illustrated in FIG. 32, the long band portion 65 is sewn on the lower portion of the body temperature regulation pack holding portion 62 on the front side of the backside portion 60 so that the center part of the band portion 65 overlaps with the backside portion 60. The entire front surface of the band portion 65 is a loop-shaped fastener cloth surface so as to enable engagement of a hook-shaped surface fastener 91 for attaching a collar belt 73 of the collar pack holder 70 to be described later. Further, similarly to the front surface, the entire rear surface of the band portion 65 is a loop-shaped fastener cloth surface, and a mesh bag 66 to be arranged under the arm can be removably attached to any position. Note that, the reason why the surface fastener cloth surfaces of the front surface and the rear surface of the band portion 65 are each a loop-shaped surface fastener is that the band portion 65 frequently comes into contact with the arms, the outer clothes, etc., and hence the feel must be comfortable, and unnecessary fastening must be prevented even when the surface fastener comes into contact with other clothing or the like. However, the present invention is not limited thereto, and the surface fastener cloth surface of the band portion 65 may be a hook-shaped surface fastener. Further, the entire rear surface need not be a surface fastener cloth surface, and a plurality of pieces of elongated surface fasteners may be arrayed in parallel to each other in the vertical direction. Still further, the shape of the band portion 65 is not limited to a single long band, and two-separated band portions 65 in a pair may be respectively sewn on the lower portions of the body temperature regulation pack holding portion 62 on both sides thereof.

At both end portions on the rear surface of the band portion 65, a pair of hook-shaped surface fasteners 92 are provided. With this, when the band portion 65 surrounds a region below the chest or the abdomen of the wearer, at the front side of the body, the hook-shaped surface fastener 92 at one end portion can engage with a loop-shaped surface fastener cloth surface on the front surface of the band portion 65 on the other end side, to thereby fix the foreside portion 50 at a position below the chest (see FIG. 28). The surface fasteners 92 are sewn at both the end portions of the band portion 65, and thus regardless of whichever end potion is provided on the front side, the end portion can be fixed to the other end side. Further, the fastening position of the surface fastener 92 can be changed as appropriate, and hence the degree of contact of the band portion 65 can be adjusted in accordance with the build of the wearer. Further, the band portion 65 is formed of a stretchable cloth which has elasticity in the longitudinal direction, and hence further adjustment is possible in accordance with build or the like.

As described above, the positions of the mesh bags 51, 61, and 66 to be arranged at the chest, the back, and under the arm can be freely selected to some extent on the rear surfaces of the body temperature regulation pack holding portions 52 and 62 and the band portion 65. Therefore, the wearer can determine not only the positions but also the number of body temperature regulation packs 1 to be arranged or the pack size by himself/herself as necessary. For example, when the torso pack holder 80 is worn under protective clothing or work clothing, the mesh bags 51, 61, or 66 may be arranged so as to avoid positions at which the equipment for working is to be installed, to thereby prevent the body temperature regulation packs 1 from interfering with the equipment and reducing safety. Further, the wearer can adjust the torso pack holder 80 in accordance with the body as a harness for climbing, and hence regardless of build, the degree of contact can be adjusted.

<Collar Pack Holder>

Next, with reference to FIGS. 25, 28 to 30, and 34, description is made of the collar pack holder 70 dedicated for wearing a body temperature regulation pack 1 around the neck. The collar pack holder 70 has a rectangular shape, and as illustrated in FIGS. 34, the collar pack holder 70 includes a mesh bag 71 dedicated to the neck, and the collar belt 73 integrally connected to both ends of the mesh bag 71.

Inside the mesh bag 71 dedicated to the neck, as illustrated in FIG. 25, a body temperature regulation pack 1'a long in the vertical direction is arranged at a position corresponding to a portion right behind the neck (center part of the mesh bag 71), and a pair of body temperature regulation packs 1'b whose lengths are smaller than that of the body temperature regulation pack 1'a are arranged at positions corresponding to lateral portions of the neck on both sides of the body temperature regulation pack 1'a. In this embodiment, as the body temperature regulation pack 1'a, a body temperature regulation pack having a height of 70 mm is used, which is obtained by coupling five elongated bag-like portions 22 in the lateral direction. As the body temperature regulation pack 1'b, a body temperature regulation pack having a height of 50 mm is used, which is obtained by coupling three elongated bag-like portions 22 in the lateral direction. By inserting the plurality of body temperature regulation packs 1', the mesh bag 71 can be folded at a boundary between the body temperature regulation pack 1'a and the body temperature regulation pack 1'b, and hence the body temperature regulation pack 1' can be easily worn in accordance with the shape around the neck. Further, by reducing the height of the body temperature regulation packs 1'b on both sides of the neck, the movement of the neck of the wearer is not interrupted. Note that, the shape and the number of the body temperature regulation packs 1' are not limited thereto, and for example, the mesh bag 71 may contain a single rectangular body temperature regulation pack 1'. Further, as illustrated in FIG. 34(c), the mesh bag 71 may contain a plurality of arrayed elongated bag members 322 of the second embodiment, which have the same length and thickness. Further, the elongated bag members 322 whose size and length are changed may be arrayed.

The material of the mesh bag 71 is mesh fabric. Therefore, when the body temperature regulation pack 1' is used for the purpose of cooling, heat released from the body can be absorbed more efficiently. Further, when the body temperature regulation pack 1' is immersed in ice water so that the phase returns to a solid phase, in the case where the mesh bag 71 is made of a mesh material, it is possible to cool the entire collar pack holder 70 by immersing the entire collar pack holder 70 in ice water without taking the body temperature regulation pack 1' out all of the time. On the other hand, when the body temperature regulation pack 1' is used for the purpose of heat retention, heat released from the body temperature regulation pack 1' can be transferred to the body more efficiently.

As illustrated in FIGS. 34, the pair of collar belts 73 are integrally fixed to both the end portions of the mesh bag 71. Further, on an end portion 72 on a side opposite to the end portion connected to the mesh bag 71, the hook-shaped surface fastener 91 is provided, which engages with the loop-shaped surface fastener cloth surface of the band portion 65. The pair of collar belts 73 have an appropriate length so that the collar belts 73 are obliquely crossed in the vicinity of the chest of the wearer so that each end portion 72 reaches the front surface of the band portion 65 and is connected through intermediation of the surface fastener 91. Further, the collar belt 73 is formed into a band shape with a stretchable cloth which has elasticity in the longitudinal direction. Because the end portion 72 of the collar belt 73 can be removably fastened to any position on the front surface of the band portion 65, and in addition, because the collar belt 73 is stretchable, the degree of contact of the collar pack holder 70 with the neck can be adjusted in accordance with the build of the wearer and the necessary degree of contact. Note that, the collar belt 73 may be fastened at the shoulder portion 64. Further, as long as freedom of movement of the neck is secured, the collar belt 73 may be looped around the neck and fixed.

<Leg Pack Holder>

Next, with reference to FIG. 35, description is made of the leg pack holder 90 dedicated for wearing the body temperature regulation pack 1 on the legs. The leg pack holder 90 has a rectangular shape, and as illustrated in FIG. 35, the leg pack holder 90 includes a mesh bag 84 dedicated to the legs, and a pair of leg belts 83 integrally connected to only one side of the mesh bag 84.

The mesh bag 84 dedicated to legs contains, as illustrated in FIG. 35, the body temperature regulation pack 1 obtained by coupling five elongated bag-like portions 22 in the lateral direction. Note that, the shape and the number of body temperature regulation packs 1 are not limited thereto. Further, the elongated bag members 322 of the second embodiment, whose size and length are changed, may be arrayed.

The material of the mesh bag 84 is mesh fabric. Therefore, when the body temperature regulation pack 1 is used for the purpose of keeping the body warm, heat released from the body temperature regulation pack 1 can be transferred to the body more efficiently. On the other hand, when the body temperature regulation pack 1 is used for the purpose of cooling the body, heat released from the body can be absorbed more efficiently. Further, when the body temperature regulation pack 1 is immersed in ice water so that the phase returns to a solid phase, in the case where the mesh bag 84 is made of a mesh material, it is possible to cool the entire leg pack holder 90 by immersing the entire leg pack holder 90 in ice water without taking the body temperature regulation pack 1 out all of the time.

As illustrated in FIG. 35, the leg belt 83 is integrally fixed to one side of the mesh bag 84. Further, an end portion 82 of the leg belt 83, which is on a side opposite to the end portion connected to the mesh bag 84, is provided with a hook-shaped surface fastener 81. Further, the surface of the leg pack holder 90 on a side which does not come into contact with the body is a loop-shaped surface fastener. The wearer wraps the leg pack holder 80 around a leg as illustrated in FIGS. 36. The surface fastener 81 of the leg belt 83 engages with the surface fastener of the leg pack holder 90. Thus, the surface fastener 81 can be removably fastened at any position. Further, the leg belt 83 is formed into a band shape with a stretchable cloth which has elasticity in the longitudinal direction. Therefore, because the end portion 82 of the band belt 83 can be removably fastened at any position on the front surface of the leg pack holder 90, and in addition, because the band belt 83 is stretchable, the degree of contact of the leg pack holder 90 with the leg can be adjusted in accordance with the build of the wearer and the necessary degree of contact.

Note that, the shape of the leg belt 83 is not limited to that in this embodiment. Alternatively, one leg belt 83 may be connected to one end of the mesh bag 84, the mesh bag 84 itself may be a surface fastener, and the surface fastener 81 of the leg belt 83 may engage to the mesh bag 84 itself. Further, the surface fastener 81 of the leg belt 83 may be a loop-shaped surface fastener, and the surface fastener of the leg pack holder 90 may be a hook-shaped surface fastener.

Note that, during outdoor work in the cold months or in a work place without a heating device, it has conventionally been relatively easy for a worker to keep his upper body warm, but efficient warming of the legs has been insufficient, and there has been a problem in that a worker feels pain with respect to cold on the feet. Therefore, the leg pack holder 90 is mainly used for the purpose of heat retention. The body temperature regulation pack 1 does not deform even in the state of the liquid phase, and hence the legs can be kept warm without unevenness. Further, it is possible to freely fold and extend the body temperature regulation pack 1 even when the solid-liquid phase change material is in the solid phase, and hence the body temperature regulation pack 1 folded along the shape of the body can be easily removed. Note that, the leg pack holder 90 may also be used for the purpose of cooling the body as described above.

In the following, description is made of advantages of the above-mentioned pack holder 2 as a whole. First, description is made with reference to the body temperature regulation pack 1 to be used for the purpose of cooling. Note that, the body temperature regulation pack 1 of the present invention has the heat insulating material 30 sealed therein together with the solid-liquid phase change material 10, and hence it is unnecessary to provide a heat insulating function to the pack holder 2. In this case, the method of causing the solid-liquid phase change material 10 inside the body temperature regulation pack 1 to return from the liquid phase to the solid phase is as follows in order of increasing length of time:

(1) immersing the solid-liquid phase change material 10 in plenty of ice water;
(2) immersing the solid-liquid phase change material 10 in running water, for example, in the river;
(3) putting the solid-liquid phase change material 10 into the refrigerator; and
(4) leaving the solid-liquid phase change material 10 at room temperature that is equal to or lower than 22°C.

In the case of a conventional pack holder having a heat insulating function, even when it is desired to cool the body temperature regulation pack while keeping the body temperature regulation pack inside the pack holder, the pack holder is bulky, and hence it is difficult to immerse the pack holder in water by the method of (1) and (2), and the pack holder itself becomes heavy with the absorbed moisture. Further, in the method of (3), because the pack holder is bulky, refrigerator space is wasted. Further, in the method of (4), this takes time to cool the body temperature regulation pack, and hence it is more desirable to adopt the method of (1), etc.

Further, when the pack holder is worn under protective clothing or the like, the entire clothing tends to become heavy because other body protective equipment such as respiratory protective equipment is added. Therefore, it is desirable for the pack holder itself to not have unnecessary weight, and to be as lightweight and unbulky as possible. Further, equipment such as respirators always need a harness, and a harness normally has a pad. Therefore, it is an unnecessary duplication to fix the heat insulating material to the pack holder itself.

In view of the above-mentioned problems, the heat insulating function is not provided to the pack holder 2 of the present invention itself, and the heat insulating material is sealed into the body temperature regulation pack 1 so that the pack holder 2 can be lightweight and not bulky. Further, because the pack holder 2 is made of a mesh material which can be quickly dried even when the pack holder 2 gets wet, the entire pack holder 2 can be immersed in ice water to be cooled. Further, a skeleton state is obtained, in which, through the mesh fabric, water and air easily flow around the entire pack holder 2. Therefore, it is possible to further improve the function recovery efficiency of the solid-liquid phase change material 10. Still further, in addition to the achievement of reduction in weight and size of the pack holder 2 itself, the position, the number, and the pack size of the body temperature regulation pack 1 can be freely selected. Thus, there is little risk of interfering with necessary equipment such as respiratory protective equipment and of reducing safety. Further, when a wearer who generally works for a certain period of time desires to specially work for a longer period of time, the mesh bag may be replaced with one containing a larger-size body temperature regulation pack 1. Thus, the object can be achieved with ease.

As a result of performing duration tests under states in which the cooling material formed of a solid-liquid phase change material was fitted, the cooling material was brought into close contact with the upper body except for the lower part of the abdomen, and clothing (mainly, clothing corresponding to the protective clothing or the like) without a heat release portion was worn thereon, the following conclusion was reached as the body sense: "At least one cold position is desired. Two cold positions on the chest and the back are more desirable than one position. Cold positions on the neck and under the arms are still more desirable than the two cold positions. Further, although it is desirable for each region to be as widely cooled as possible, a long-lasting heat absorption effect is more desirable than quick melting even if the heat absorption area is narrow." Therefore, the pack holder 2 of the present invention has a configuration in which, in order to achieve these objects, body temperature regulation packs 1 of any sizes can be arranged at any regions.

Description has been made above of the preferred embodiments of the present invention, but the present invention is not limited to the above-mentioned embodiments, and various changes can be made thereto within the scope of claims.

Reference Signs List

1 body temperature regulation pack

2 pack holder

10 solid-liquid phase change material

20 inner pack

22 elongated bag-like portion

30 heat insulating material

40 outer pack

50 foreside portion

60 backside portion

65 band portion

70 collar pack holder

80 torso pack holder

90 leg pack holder

100 body temperature regulation pack

322 elongated bag member

## Claims

**1.** A body temperature regulation pack having a solid-liquid phase change material filled therein, the body temperature regulation pack comprising a plurality of bag members arranged in parallel to each other, wherein the plurality of bag members are each filled with the solid-liquid phase change material without gaps.

**2.** A body temperature regulation pack according to claim 1, wherein the plurality of bag members each have an elongated cylindrical shape.

**3.** A body temperature regulation pack according to claim 1 or 2, wherein the plurality of bag members each comprise an independent elongated bag member.

**4.** A body temperature regulation pack according to claim 1 or 2, wherein the plurality of bag members comprise elongated bag-like portions formed by bonding together surrounding edge portions of a pair of inner sheets, and welding a plurality of positions in parallel to each other.

**5.** A body temperature regulation pack according to any one of claims 1 to 4, wherein the body temperature regulation pack has one surface covered with a heat insulating material.

**6.** A body temperature regulation pack according to any one of claims 1 to 5, wherein the solid-liquid phase change material contains any one of sodium sulfate, sodium acetate, and normal paraffin.

**7.** A torso pack holder for holding the body temperature regulation pack according to any one of claims 1 to 6 onto a body side, the torso pack holder being configured to enable removable attaching of the body temperature regulation pack at any position.

**8.** A torso pack holder according to claim 7,
wherein the torso pack holder comprises a foreside portion for covering a chest of a wearer, and a backside portion for covering a back of the wearer, and
wherein the foreside portion and the backside portion are disengageably connected to each other.

**9.** A torso pack holder according to claim 8, wherein the foreside portion and the backside portion are connected to each other with a stretchable band-like member.

**10.** A collar pack holder for removably wearing the body temperature regulation pack according to any one of claims 1 to 6 around a neck.

**11.** A leg pack holder for removably wearing the body temperature regulation pack according to any one of claims 1 to 6 to a leg.

[FIG. 1]

T
T
1
T
40
T
42
43a
43b

[FIG. 2]

40
41
22
21a
43a
43b
42
23
10
20
21b
30

[FIG. 3]

26(25b)
23
20
21a(b)
25a
25a
25c
22

[FIG. 4]

26(25b)
23
20
21a(b)
10
25a
25a
25c
22

25a
23
22
25a
10

[FIG. 5]

24
25b
23
10
25a
25a
20
25c
22

[FIG. 6]

10
23
22
20
25a
25a
25c

[FIG. 7]

10
23
22
25a
10
22
25a
23

[FIG. 8]

30
20
10
23
22

[FIG. 9]

42
43a
41
30
40
43b
10
22

[FIG. 10]

30
42
40
41
10
23
22

[FIG. 11]

40
10
22
23
30
42
41

[FIG. 12]

1
1
1

[FIG. 13]

326
322
325a
322
325a

[FIG. 14]

325b
322
325a
325a(b)
322
325b
325a
322
325a(b)
322

[FIG. 15]

325b
322
325a
325b
325a
322

[FIG. 16]

322
27
325a(b)
28
29
322
27
29

[FIG. 17]

29
27
28
[FIG. 18]
100
27
30
10
322
[FIG. 19]
100
41
27
30
40
42
43a
43b
10
322
[FIG. 20]
100
27
30
42
40
41
10
322

[FIG. 21]

322
27
30´
30´a
+
30´b

[FIG. 22]

22″
22′

[FIG. 23]

11
13
13
12
12
H
14
13
13
11

[FIG. 24]

1
h
11
1
11
1
11
1
11
1
11

[FIG. 25]

1´a
1´b
1´b
1´

[FIG. 26]

51
1

[FIG. 27]

(a)
51
1
FRONT
(b)
97b
51
REAR
97b

[FIG. 28]

2
70
64
64
50
52
80
73
73
65

[FIG. 29]

2
70
62
80
60
65

[FIG. 30]

70
2
60
51
62
52
61
80
50
65
66

[FIG. 31]

50
52
97a
97a
97a
97a
97a
97a
97a
(b)
59
51
59

50
52
95a
95a
(a)

[FIG. 32]

60
64
62
65

95b
95b
64
62
69
61
94a
92
66
66
92
65
94a
94a
94a
69

[FIG. 33]

80
55
60
50

[FIG. 34]

(a)
70
71
73
72
91
91

(b)
70
71
73

(c)
70
71
73
72
91
91

[FIG. 35]

83
1
90
81
84
90

[FIG. 36]

(a)
105
102
103
103
103
104
(b)
102
103
103
104

[FIG. 37]

101

[FIG. 38]

101 101
106
BODY SIDE
COUNTER-BODY SIDE
103(104)
102

[FIG. 39]

202
201
201

[FIG. 40]

(a)
201
206
208a
208b
207
208

(b)
201
NON-BODY SIDE
206
208
207
BODY SIDE

(c)
NON-BODY SIDE
201
206
208
BODY SIDE

[FIG. 41]

101(201)
101´(201´)

**INTERNATIONAL SEARCH REPORT**

International application No.
PCT/JP2011/079611

A. CLASSIFICATION OF SUBJECT MATTER
*F25D3/00*(2006.01)i, *A41D13/00*(2006.01)i

According to International Patent Classification (IPC) or to both national classification and IPC

B. FIELDS SEARCHED

Minimum documentation searched (classification system followed by classification symbols)
F25D3/00, A41D13/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2009-127147 A (Kabushiki Kaisha Aguri Soken), 11 June 2009 (11.06.2009), claims; paragraphs [0001] to [0040]; fig. 1 to 5 | 1-11 |
| Y | US 6185742 B1 (Brian Doherty), 13 February 2001 (13.02.2001), fig. 1 to 3; columns 1 to 4 | 1-11 |
| Y | JP 2009-119197 A (Shin KIYOKAWA), 04 June 2009 (04.06.2009), paragraphs [0065] to [0072] | 6-11 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 January, 2012 (11.01.12) | 24 January, 2012 (24.01.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2011/079611

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-169858 A (Aitoz Corp.), 05 July 2007 (05.07.2007), claims; paragraphs [0001] to [0021]; fig. 1 to 3 | 7-11 |
| Y | JP 2002-309414 A (Aichi Corp.), 23 October 2002 (23.10.2002), paragraphs [0025] to [0031]; fig. 3 to 4 | 8-11 |
| Y | JP 8-199408 A (Ashimori Industry Co., Ltd.), 06 August 1996 (06.08.1996), claims; paragraphs [0001] to [0037]; fig. 1 to 2 | 8-11 |
| Y | JP 2007-528945 A (Royal Melbourne Institute of Technology), 18 October 2007 (18.10.2007), paragraphs [0045] to [0046] | 11 |
| A | JP 2003-328213 A (Aichi Corp.), 19 November 2003 (19.11.2003), entire text; all drawings | 1-11 |
| A | JP 2010-255152 A (Alpha Technical Research Co., Ltd.), 11 November 2010 (11.11.2010), entire text; all drawings | 1-11 |

Form PCT/ISA/210 (continuation of second sheet) (July 2009)

**INTERNATIONAL SEARCH REPORT**
Information on patent family members

International application No.
PCT/JP2011/079611

| | | | |
|---|---|---|---|
| JP 2009-127147 A | 2009.06.11 | (Family: none) | |
| US 6185742 B1 | 2001.02.13 | (Family: none) | |
| JP 2009-119197 A | 2009.06.04 | WO 2009/054081 A1 | 2009.04.30 |
| JP 2007-169858 A | 2007.07.05 | (Family: none) | |
| JP 2002-309414 A | 2002.10.23 | (Family: none) | |
| JP 8-199408 A | 1996.08.06 | (Family: none) | |
| JP 2007-528945 A | 2007.10.18 | US 2006/0276089 A1 | 2006.12.07 |
| | | WO 2005/006896 A1 | 2005.01.27 |
| | | CA 2532560 A1 | 2005.01.27 |
| | | AU 2004257352 A1 | 2005.01.27 |
| JP 2003-328213 A | 2003.11.19 | (Family: none) | |
| JP 2010-255152 A | 2010.11.11 | (Family: none) | |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003328213 A
- JP 2010255152 A